(19) Europäisches Patentamt
European Patent Office
Office européen des brevets

(11) EP 1 156 050 B1

(12) EUROPEAN PATENT SPECIFICATION

(45) Date of publication and mention of the grant of the patent:
**18.02.2004 Bulletin 2004/08**

(51) Int Cl.[7]: **C07D 513/04**, C07D 471/04, C07D 491/04, A61K 31/40

(21) Application number: **01203116.7**

(22) Date of filing: **16.04.1999**

(54) **Substituted tricyclics useful in sPLA2 induced diseases**

Substituierte tricyclische Verbindungen und deren Verwendung zur Behandlung von sPLA2-bedingten Erkrankungen

Composés tricycliques substitués pour le traitement des maladies induites par sPLA2

(84) Designated Contracting States:
**AT BE CH CY DE DK ES FI FR GB GR IE IT LI LU NL PT SE**
Designated Extension States:
**AL LT LV RO SI**

(30) Priority: **17.04.1998 US 62165**

(43) Date of publication of application:
**21.11.2001 Bulletin 2001/47**

(62) Document number(s) of the earlier application(s) in accordance with Art. 76 EPC:
**99302969.3 / 0 950 661**

(73) Proprietor: **ELI LILLY AND COMPANY**
**Indianapolis, Indiana 46285 (US)**

(72) Inventors:
- **Bach, Nicholas James**
  **Indianapolis 46217 (US)**
- **Bastian, Jolie Anne**
  **Beech Grove, Indiana 46107 (US)**
- **Beight, Douglas Wade**
  **Frankfort, Indiana 46041 (US)**
- **Kinnick, Michael Dean**
  **Indianapolis, Indiana 46217 (US)**
- **Martinelli, Michael John**
  **Zionsville, Indiana 46077 (US)**
- **Mihelich, Edward David**
  **Carmel, Indiana 46032 (US)**
- **Morlin, John Michael, Jr.**
  **Brownsburg, Indiana 46112 (US)**
- **Sall, Daniel Jon**
  **Greenwood, Indiana 46143 (US)**
- **Sawyer, Jason Scott**
  **Indianapolis, Indiana 46220 (US)**
- **Smith, Edward C. R.**
  **Fishers, Indiana 46038 (US)**
- **Suarez, Tulio**
  **Greenwood, Indiana 46143 (US)**
- **Wang, Quiping**
  **Fishers, Indiana 46038 (US)**
- **Wilson, Thomas Michael**
  **Speedway, Indiana 46224 (US)**

(74) Representative: **Pritchard, Judith et al**
**Lilly Research Centre,**
**Erl Wood Manor**
**Windlesham, Surrey GU20 6PH (GB)**

(56) References cited:
**EP-A- 0 779 271**
**EP-A- 0 839 806**
**WO-A-96/03383**
**WO-A-98/18464**
**WO-A-99/16453**
**WO-A-99/25340**
**US-A- 3 979 391**

- **GARRATT P J ET AL: "MAPPING THE MELATONIN RECEPTOR. 2. SYNTHESIS AND BIOLOGICAL ACTIVITY OF INDOLE DERIVED MELATONIN ANALOGUES WITH RESTRICTED CONFORMATIONS OF THE C-3 AMIDOETHANE SIDE CHAIN" BIOORGANIC & MEDICINAL CHEMISTRY LETTERS, vol. 4, no. 13, 1 January 1994 (1994-01-01), pages 1559-1564, XP002054522**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 156 050 B1

## Description

This invention relates to novel substituted tricyclic organic compounds useful for inhibiting $sPLA_2$ mediated release of fatty acids for conditions such as septic shock.

The structure and physical properties of human non-pancreatic secretory phospholipase $A_2$ (hereinafter called, "$sPLA_2$") has been thoroughly described in two articles, namely, "Cloning and Recombinant Expression of Phospholipase $A_2$ Present in Rheumatoid Arthritic Synovial Fluid" by Seilhamer, Jeffrey J.; Pruzanski, Waldemar; Vadas Peter; Plant, Shelley; Miller, Judy A.; Kloss, Jean; and Johnson, Lorin K.; The Journal of Biological Chemistry, Vol. 264, No. 10, Issue of April 5, pp. 5335-5338, 1989; and "Structure and Properties of a Human Non-pancreatic Phospholipase $A_2$" by Kramer, Ruth M.; Hession, Catherine; Johansen, Berit; Hayes, Gretchen; McGray, Paula; Chow, E. Pingchang; Tizard, Richard; and Pepinsky, R. Blake; The Journal of Biological Chemistry, Vol. 264, No. 10, Issue of April 5, pp. 5768-5775, 1989; the disclosures of which are incorporated herein by reference.

It is believed that $sPLA_2$ is a rate limiting enzyme in the arachidonic acid cascade which hydrolyzes membrane phospholipids. Thus, it is important to develop compounds which inhibit $sPLA_2$ mediated release of fatty acids (e.g., arachidonic acid). Such compounds would be of value in general treatment of conditions induced and/or maintained by overproduction of $sPLA_2$ such as septic shock, adult respiratory distress syndrome, pancreatitis, trauma-induced shock, bronchial asthma, allergic rhinitis, rheumatoid arthritis, etc.

It is desirable to develop new compounds and treatments for $sPLA_2$ induced diseases.

Alexander, et al., United States Patent Nos. 3,939,177 and 3,979,391, disclose 1,2,3,4-tetrahydrocarbazoles useful as antibacterial agents.

This invention provides tricyclic compounds as defined in Claim 1 below, which compounds are encompassed within the structure depicted in the general formula (I) below:

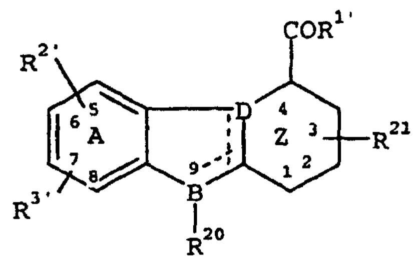

(I)

wherein;

| | |
|---|---|
| A | is phenyl or pyridyl wherein the nitrogen is at the 5-6-, 7- or 8-position; |
| one of B or D | is nitrogen and the other is carbon; |
| Z | is cyclohexenyl, phenyl, pyridyl, wherein the nitrogen is at the 1-, 2-, or 3-position, or a 6-membered heterocyclic ring having one heteroatom selected from the group consisting of sulfur or oxygen at the 1-, 2- or 3-position, and nitrogen at the 1-, 2-, 3- or 4-position; |
| ---- | is a double or single bond; |
| $R^{20}$ | is selected from groups (a), (b) and (c) where; |

(a) is -($C_5$-$C_{20}$)alkyl, -($C_5$-$C_{20}$)alkenyl, ($C_5$-$C_{20}$)alkynyl, carbocyclic radicals, or heterocyclic radicals, or

(b) is a member of (a) substituted with one or more independently selected non-interfering substituents; or

(c) is the group -(L)-$R^{80}$; where, -(L)- is a divalent linking group of 1 to 12 atoms selected from carbon, hydrogen, oxygen, nitrogen, and sulfur; wherein the combination of atoms in -(L)- are selected from the group consisting of (i) carbon and hydrogen only, (ii) one sulfur only, (iii) one oxygen only, (iv) one or two nitrogen and hydrogen only, (v) carbon, hydrogen, and one sulfur only, and (vi) and carbon, hydrogen, and oxygen only; and where $R^{80}$ is a group selected from (a) or (b);

| | |
|---|---|
| $R^{21}$ | is a non-interfering substituent; |
| R1' | is $-NHNH_2$, $-NH_2$ or $-CONH_2$; |
| $R^{2'}$ | is selected from the group consisting of -OH, and $-O(CH_2)_tR^{5'}$ where |
| $R^{5'}$ | is H, -CN, $-NH_2$, $-CONH_2$, $-CONR^9R^{10}$ $-NHSO_2R^{15}$; $-CONHSO_2R^{15}$, where $R^{15}$ is -($C_1$-$C_6$)alkyl or |

-$CF_3$; phenyl or phenyl substituted with -$CO_2H$ or -$CO_2(C_1$-$C_4)$alkyl; and - $(L_a)$ - (acidic group), wherein -$(L_a)$- is an acid linker having an acid linker length of 1 to 7 and t is 1-5;

$R^{3'}$ is selected from non-interfering substituent, carbocyclic radicals, carbocyclic radicals substituted with non-interfering substituents, heterocyclic radicals, and heterocyclic radicals substituted with non-interfering substituents; or a pharmaceutically acceptable racemate, solvate, tautomer, optical isomer, prodrug derivative or salt thereof;

provided that one of A or Z is a heterocyclic ring; and when D is nitrogen, the heteroatom of Z is selected from the group consisting of sulfur or oxygen at the 1-, 2- or 3-position and nitrogen at the 1-, 2-, 3- or 4-position.

These substituted tricyclics are effective in inhibiting human $sPLA_2$ mediated release of fatty acids.

This invention is also a pharmaceutical formulation comprising a compound of Claim 1 in association with one or more pharmaceutically acceptable diluents, carriers and excipients.

This invention is also the use of a compound as claimed in Claim 1 for the manufacture of a medicament for inhibiting $sPLA_2$ in a mammal in need of such treatment.

According to a further aspect of the present invention, there is provided the use of a compound as claimed in Claim 1 for the manufacture of a medicament for selectively inhibiting $sPLA_2$ in a mammal in need of such treatment

This invention also provides the use of a compound as claimed in Claim 1 for the manufacture of a medicament for alleviating the pathological effects of sepsis, septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis and related diseases which comprises administering to a mammal in need of such treatment a therapeutically effective amount of the compound of formula I in an amount sufficient to inhibit $sPLA_2$ mediated release of fatty acid and to thereby inhibit or prevent the arachidonic acid cascade and its deleterious products.

Other objects, features and advantages of the present invention will become apparent from the subsequent description and the appended claims.

Definitions:

As used herein, the term, "alkyl" by itself or as part of another substituent means, unless otherwise defined, a straight or branched chain monovalent hydrocarbon radical such as methyl, ethyl, n-propyl, isopropyl, n-butyl, tertiary butyl, isobutyl, sec-butyl tert butyl, n-pentyl, isopentyl, neopentyl, heptyl, hexyl, octyl, nonyl, decyl, undecyl, dodecyl, tridecyl, tetradecyl and the like. The term "alkyl" includes -$(C_1$-$C_2)$alkyl, -$(C_1$-$C_4)$alkyl, -$(C_1$-$C_6)$alkyl, -$(C_5$-$C_{14})$alkyl, and -$(C_1$-$C_{10})$alkyl.

The term "alkenyl" as used herein represents an olefinically unsaturated branched or linear group having at least one double bond. Examples of such groups include radicals such as vinyl, allyl, 2-butenyl, 3-butenyl, 2-pentenyl, 3-pentenyl, 4-pentenyl, 2-hexenyl, 3-hexenyl, 4-hexenyl, 5-hexenyl, 2-heptenyl, 3-heptenyl, 4-heptenyl, 5-heptenyl, 6-heptenyl as well as dienes and trienes of straight and branched chains.

The term "alkynyl" denotes such radicals as ethynyl, propynyl, butynyl, pentynyl, hexynyl, heptynyl as well as di- and tri-ynes.

The term "halo" means chloro, fluoro, bromo or iodo.

The term "-$(C_1$-$C_4)$alkoxy", as used herein, denotes a group such as methoxy, ethoxy, n-propoxy, isopropoxy, n-butoxy, t-butoxy and like groups, attached to the remainder of the molecule by the oxygen atom.

The term "phenyl$(C_1$-$C_4)$alkyl" refers to a straight or branched chain alkyl group having from one to four carbon atoms attached to a phenyl ring which chain is attached to the remainder of the molecule. Typical phenylalkyl groups include benzyl, phenylethyl, phenylpropyl, phenylisopropyl, and phenylbutyl.

The term "-$(C_1$-$C_4)$alkylthio" defines a straight or branched alkyl chain having one to four carbon atoms attached to the remainder of the molecule by a sulfur atom. Typical -$(C_1$-$C_4)$alkylthio groups include methylthio, ethylthio, propylthio, butylthio and the like.

The term "-($C_3$-$C_{14}$)cycloalkyl" includes groups such as cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl and the like. The term "-($C_3$-$C_{14}$)cycloalkyl" includes and -($C_3$-$C_7$)cycloalkyl.

The term, "heterocyclic radical", refers to radicals derived from monocyclic or polycyclic, saturated or unsaturated, substituted or unsubstituted heterocyclic nuclei having 5 to 14 ring atoms and containing from 1 to 3 hetero atoms selected from the group consisting of nitrogen, oxygen or sulfur. Typical heterocyclic radicals are pyridyl, thienyl, fluorenyl, pyrrolyl, furanyl, thiophenyl, pyrazolyl, imidazolyl, phenylimidazolyl, triazolyl, isoxazolyl, oxazolyl, thiazolyl, thiadiazolyl, indolyl, carbazolyl, norharmanyl, azaindolyl, benzofuranyl, dibenzofuranyl, thianaphtheneyl, dibenzothiophenyl, indazolyl, imidazo(1.2-A)pyridinyl, benzotriazolyl, anthranilyl, 1,2-benzisoxazolyl, benzoxazolyl, benzothiazolyl, purinyl, pryidinyl, dipyridylyl, phenylpyridinyl, benzylpyridinyl, pyrimidinyl, phenylpyrimidinyl, pyrazinyl, 1,3,5-triazinyl, quinolinyl, phthalazinyl, quinazolinyl, and quinoxalinyl.

The term "carbocyclic radical" refers to radicals derived from a saturated or unsaturated, substituted or unsubstituted 5 to 14 membered organic nucleus whose ring forming atoms (other than hydrogen) are solely carbon atoms. Typical carbocyclic radicals are cycloalkyl, cycloalkenyl, phenyl, naphthyl, norbornanyl, bicycloheptadienyl, tolulyl, xylenyl, indenyl, stilbenyl, terphenylyl, diphenylethylenyl, phenylcyclohexeyl, acenaphthylenyl, and anthracenyl, biphenyl, bibenzylyl and related bibenzylyl homologues represented by the formula (bb),

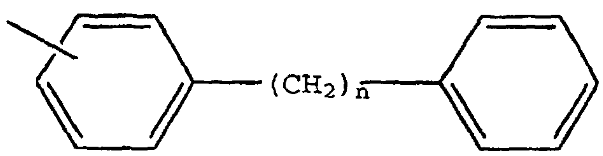

(bb)

where n is an integer from 1 to 8.

The term, "non-interfering substituent", refers to radicals suitable for substitution at positions 1, 2, 3, 7 and/or 8 on the tricyclic nucleus (as depicted in Formula III) and radical(s) suitable for substitution on the heterocyclic radical and carbocyclic radical as defined above. Illustrative non-interfering radicals are hydrogen, -($C_1$-$C_{14}$)alkyl, -($C_2$-$C_6$)alkenyl, -($C_2$-$C_6$)alkynyl, -($C_7$-$C_{12}$)aralkyl, -($C_7$-$C_{12}$)alkaryl, -($C_3$-$C_8$)cycloalkyl, -($C_3$-$C_8$)cycloalkenyl, phenyl, tolulyl, xylenyl, biphenyl, -($C_1$-$C_6$)alkoxy, -($C_2$-$C_6$)alkenyloxy, -($C_2$-$C_6$)alkynyloxy, -($C_1$-$C_{12}$)alkoxyalkyl, -($C_1$-$C_{12}$)alkoxyalkyloxy, -($C_1$-$C_{12}$)alkylcarbonyl, -($C_1$-$C_{12}$)alkylcarbonylamino, -($C_1$-$C_{12}$)alkoxyamino, -($C_1$-$C_{12}$)alkoxyaminocarbonyl, -($C_1$-$C_{12}$)alkylamino, -($C_1$-$C_6$)alkylthio, -($C_1$-$C_{12}$)alkylthiocarbonyl, -($C_1$-$C_6$)alkylsulfinyl, -($C_1$-$C_6$)alkylsulfonyl, -($C_1$-$C_6$)haloalkoxy, -($C_1$-$C_6$)haloalkylsulfonyl, -($C_1$-$C_6$)haloalkyl, -($C_1$-$C_6$)hydroxyalkyl, -$(CH_2)_n$CN, -$(CH_2)_n$$NR^9R^{10}$, -C(O)O($C_1$-$C_6$alkyl), -$(CH_2)_n$O($C_1$-$C_6$ alkyl), benzyloxy, phenoxy, phenylthio; -($CONHSO_2$)$R^{15}$, where $R^{15}$ is -($C_1$-$C_6$)alkyl; -$CF_3$, naphthyl or -$(CH_2)_s$phenyl where s is 0-5;

-CHO, -$CF_3$, -$OCF_3$, pyridyl, amino, amidino, halo, carbamyl, carboxyl, carbalkoxy, -$(CH_2)_nCO_2H$, cyano, cyanoguanidinyl, guanidino, hydrazide, hydrazino, hydrazido, hydroxy, hydroxyamino, nitro, phosphono, -$SO_3H$, thioacetal, thiocarbonyl, furyl, thiophenyl -$COR^9$, -$CONR^9R^{10}$, -$NR^9R^{10}$, - $NCHCOR^9$, -$SO_2R^9$, -$OR^9$, -$SR^9$, $CH_2SO_2R^9$, tetrazolyl or tetrazolyl substituted with -($C_1$-$C_6$)alkyl, phenyl or -($C_1$-$C_4$)alkylphenyl, -$(CH_2)_n$OSi($C_1$-$C_6$)alkyl and ($C_1$-$C_6$)alkylcarbonyl; where n is from 1 to 8 and $R^9$ and $R^{10}$ are independently hydrogen, -$CF_3$, phenyl, -($C_1$-$C_4$)alkyl, -($C_1$-$C_4$)alkylphenyl or -phenyl($C_1$-$C_4$)alkyl

The term, "acidic group" means an organic group which when attached to a tricyclic nucleus, through suitable linking atoms (hereinafter defined as the "acid linker"), acts as a proton donor capable of hydrogen bonding. Illustrative of an acidic group are the following:

-$CO_2H$,

-5-tetrazolyl,

-$SO_3H$,

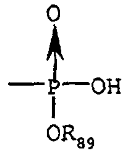

,

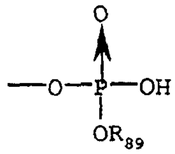

,

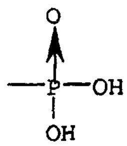

,

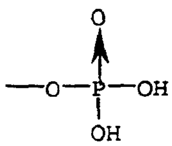

,

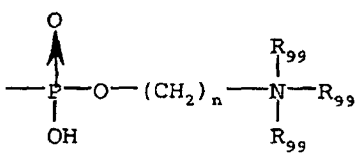

,

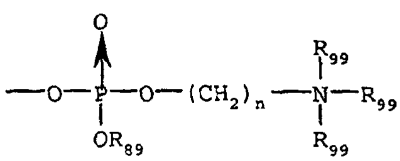

,

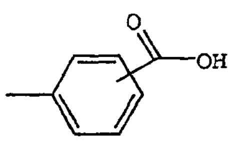

,

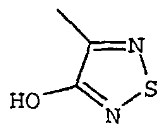 ;

where n is 1 to 8, $R_{89}$ is a metal or -($C_1$-$C_{10}$)alkyl, and $R_{99}$ is hydrogen or -($C_1$-$C_{10}$)alkyl.

The words, "acid linker" refer to a divalent linking group symbolized as, -($L_a$)-, which has the function of joining the 5 or 6 position of the tricyclic nucleus to an acidic group in the general relationship:

(tricyclic nucleus) -($L_a$)- Acidic Group

The words, "acid linker length", refer to the number of atoms (excluding hydrogen) in the shortest chain of the linking group -($L_a$)- that connects the 5 or 6 position of the tricyclic nucleus with the acidic group. The presence of a carbocyclic ring in -($L_a$)- counts as the number of atoms approximately equivalent to the calculated diameter of the carbocyclic ring. Thus, a benzene or cyclohexane ring in the acid linker counts as 2 atoms in calculating the length of -($L_a$)-. Illustrative acid linker groups are;

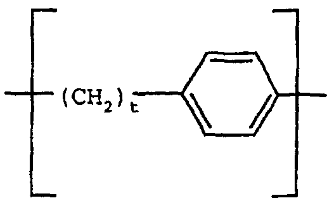
(a)

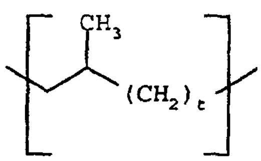
(b)

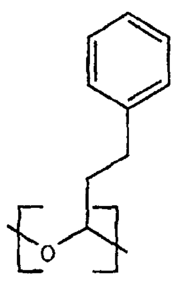
(c)

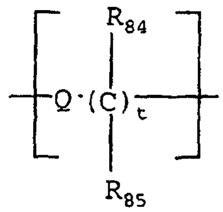

(d)

where t is 1 to 5, Q is selected from the group $-(CH_2)-$, -O-, -NH-, and -S-, and $R_{84}$ and $R_{85}$ are each independently selected from hydrogen, $-(C_1-C_{10})$alkyl, aryl, $-(C_1-C_{10})$alkaryl, $-(C_1-C_{10})$aralkyl, carboxy, carbalkoxy, and halo, when t is one (1), groups (a), (b), (c) and (d) have acid linker lengths of 3, 3, 2, and 2, respectively.

The skilled artisan will appreciate that the position of the double bond in the center 5-membered ring depends on the position of the nitrogen atom as depicted below.

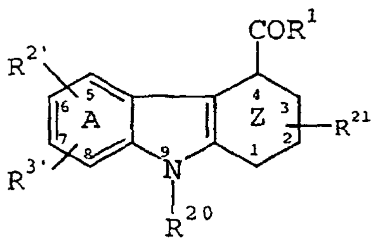

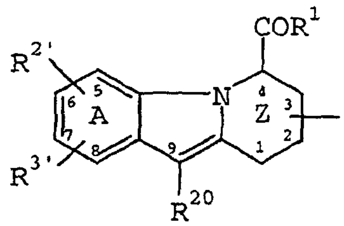

The salts of the above tricyclics are an additional aspect of the invention. In those instances where the compounds of the invention possess acidic functional groups various salts may be formed which are more water soluble and physiologically suitable than the parent compound. Representative pharmaceutically acceptable salts include but are not limited to the alkali and alkaline earth salts such as lithium, sodium, potassium, calcium, magnesium, aluminum and the like. Salts are conveniently prepared from the free acid by treating the acid in solution with a base or by exposing the acid to an ion exchange resin.

Included within the definition of pharmaceutically acceptable salts are the relatively non-toxic, inorganic and organic base addition salts of compounds of the present invention, for example, ammonium, quaternary ammonium, and amine cations, derived from nitrogenous bases of sufficient basicity to form salts with the compounds of this invention (see, for example, S. M. Berge, et al., "Pharmaceutical Salts," J. Phar. Sci., 66: 1-19 (1977)).

Compounds of the invention may have chiral centers and exist in optically active forms. R- and S-isomers and racemic mixtures are contemplated by this invention. A particular stereoisomer may be prepared by known methods using stereospecific reactions with starting materials containing asymmetric centers already resolved or, alternatively, by subsequent resolution of mixtures of stereoisomers using known methods.

The term "acid protecting group" is used herein as it is frequently used in synthetic organic chemistry, to refer to a group which will prevent an acid group from participating in a reaction carried out on some other functional group in the molecule, but which can be removed when it is desired to do so. Such groups are discussed by T. W. Greene in chapter 5 of Protective Groups in Organic Synthesis, John Wiley and Sons, New York, 1981, incorporated herein by reference in its entirety. Examples of acid protecting groups include ester or amide derivatives of the acid group, such as, methyl, methoxymethyl, methyl-thiomethyl, tetrahydropyranyl, methoxyethoxymethyl, benzyloxymethyl, phenyl, aryl, ethyl, 2,2,2-trichloroethyl, 2-methylthioethyl, t-butyl, cyclopentyl, triphenylmethyl, diphenylmethyl, benzyl, trimethylsilyl, N,N-dimethyl, pyrrolidinyl, piperidinyl, or o-nitroanilide. A preferred acid-protecting group is methyl.

Prodrugs are derivatives of the compounds of the invention which have chemically or metabolically cleavable groups and become by solvolysis or under physiological conditions the compounds of the invention which are pharmaceutically active in vivo. Derivatives of the compounds of this invention have activity in both their acid and base derivative forms, but the acid derivative form often offers advantages of solubility, tissue compatibility, or delayed release in a mammalian organism (see, Bundgard, H., Design of Prodrugs, pp. 7-9, 21-24, Elsevier, Amsterdam 1985). Prodrugs include acid derivatives, such as, esters prepared by reaction of the parent acidic compound with a suitable alcohol, or amides prepared by reaction of the parent acid compound with a suitable amine. Simple aliphatic esters (e.g., methyl, ethyl, propyl, isopropyl, butyl, sec-butyl, tert-butyl) or aromatic esters derived from acidic groups pendent on the compounds of this invention are preferred prodrugs. Other preferred esters include morpholinoethyloxy, diethylglycolamide and diethylaminocarbonylmethoxy. In some cases it is desirable to prepare double ester type prodrugs such as (acyloxy)

alkyl esters or ((alkoxycarbonyl)oxy)alkyl esters.

Compounds of formula I which comprise the present invention include:

2-[4-oxo-5-carboxamido-9-(2-methylbenzyl)-*9H*-pyrido[3,4-*b*]indolyl]acetic acid;

2-[4-oxo-5-carboxamido-9-(3-methylbenzyl)-*9H*-pyrido[3,4-*b*]indolyl]acetic acid;

2-[4-oxo-5-carboxamido-9-(4-methylbenzyl)-*9H*-pyrido[3,4-*b*]indolyl]acetic acid;

2-[4-oxo-5-carboxamido-9-(4-*tert*-butylbenzyl)-*9H*-pyrido[3,4-*b*]indolyl]acetic acid;

2-[4-oxo-5-carboxamido-9-pentafluorobenzyl-*9H*-pyrido[3,4-*b*]indolyl]acetic acid;

2-[4-oxo-5-carboxamido-9-(2-fluorobenzyl)-*9H*-pyrido[3,4-*b*]indolyl]acetic acid;

2-[4-oxo-5-carboxamido-9-(3-fluorobenzyl)-*9H*-pyrido[3,4-*b*]indolyl]acetic acid;

2-[4-oxo-5-carboxamido-9-(4-fluorobenzyl)-*9H*-pyrido[3,4-*b*]indolyl]acetic acid;

2-[4-oxo-5-carboxamido-9-(2,6-difluorobenzyl)-*9H*-pyrido[3,4-*b*]indolyl]acetic acid;

2-[4-oxo-5-carboxamido-9-(3,4-difluorobenzyl)-*9H*-pyrido[3,4-*b*]indolyl]acetic acid;

2-[4-oxo-5-carboxamido-9-(2,5-difluorobenzyl)-*9H*-pyrido[3,4-*b*]indolyl]acetic acid;

2-[4-oxo-5-carboxamido-9-(3,5-difluorobenzyl)-*9H*-pyrido[3,4-*b*]indolyl]acetic acid;

2-[4-oxo-5-carboxamido-9-(2,4-difluorobenzyl)-*9H*-pyrido[3,4-*b*]indolyl)acetic acid;

2-[4-oxo-5-carboxamido-9-(2,3-difluorobenzyl)-*9H*-pyrido[3,4-*b*]indolyl]acetic acid;

2-[4-oxo-5-carboxamido-9-[2-(trifluoromethyl)benzyl]-*9H*-pyrido[3,4-*b*]indolyl]acetic acid;

2-[4-oxo-5-carboxamido-9-[3-(trifluoromethyl)benzyl]-*9H*-pyrido[3,4-*b*]indolyl]acetic acid;

2-[4-oxo-5-carboxamido-9-[4-(trifluoromethyl)benzyl]-*9H*-pyrido[3,4-*b*]indolyl]acetic acid;

2-[4-oxo-5-carboxamido-9-[3,5-bis(trifluoromethyl)benzyl]-9*H*-pyrido[3,4-*b*] indolyl]acetic acid;

2-[4-oxo-5-carboxamido-9-[2,4-bis(trifluoromethyl)benzyl]-*9H*-pyrido[3,4-*b*]indolyl]acetic acid;

2-[4-oxo-5-carboxamido-9-(a-methylnaphthyl)-*9H*-pyrido[3,4-b]indolyl]acetic acid;

2-[4-oxo-5-carboxamido-9-(b-methylnaphthyl)-*9H*-pyrido[3,4-*b*]indolyl]acetic acid;

2-[4-oxo-5-carboxamido-9-(3,5-dimethylbenzyl)-*9H*-pyrido[3,4-*b*]indolyl]acetic acid;

2-[4-oxo-5-carboxamido-9-(2,4-dimethylbenzyl)-*9H*-pyrido[3,4-b]indolyl]acetic acid;

2-[4-oxo-5-carboxamido-9-(2-phenylbenzyl)-9H-pyrido[3,4-*b*]indolyl]acetic acid;

2-[4-oxo-5-carboxamido-9-(3-phenylbenzyl)-*9H*-pyrido[3,4-b]indolyl]acetic acid;

2-[4-oxo-5-carboxamido-9-(4-phenylbenzyl)-*9H*-pyrido[3,4-*b*]indolyl]acetic acid;

2-[4-oxo-5-carboxamido-9-(1-fluorenylmethyl)-*9H*-pyrido[3,4-*b*]indolyl]acetic acid;

2-[4-oxo-5-carboxamido-9-(2-fluoro-3-methylbenzyl)-9H-pyrido[3,4-*b*]indolyl]acetic acid;

2-[4-oxo-5-carboxamido-9-(3-benzoylbenzyl)-*9H*-pyrido[3,4-*b*]indolyl]acetic acid;

2-[4-oxo-5-carboxamido-9-(2-phenoxybenzyl)-*9H*-pyrido[3,4-*b*]indolyl]acetic acid;

2-[4-oxo-5-carboxamido-9-(3-phenoxybenzyl)-*9H*-pyrido[3,4-*b*]indolyl]acetic acid;

2-[4-oxo-5-carboxamido-9-(4-phenoxybenzyl)-*9H*-pyrido[3,4-*b*]indolyl]acetic acid;

2-[4-oxo-5-carboxamido-9-[3-[2-(fluorophenoxy)benzyl]]-*9H*-pyrido[3,4-*b*]indolyl]acetic acid;

2-[4-oxo-5-carboxamido-9-[3-[4-(fluorophenoxy)benzyl]]-*9H*-pyrido[3,4-*b*]indolyl]acetic acid;

2-[4-oxo-5-carboxamido-9-[2-fluoro-3-(trifluoromethyl)benzyl]-*9H*-pyrido[3,4-*b*]indolyl]acetic acid;

2-[4-oxo-5-carboxamido-9-[2-fluoro-4-(trifluoromethyl)benzyl]-*9H*-pyrido[3,4-*b*]indolyl]acetic acid;

2-[4-oxo-5-carboxamido-9-[2-fluoro-5-(trifluoromethyl)benzyl]-*9H*-pyrido[3,4-*b*]indolyl]acetic acid;

2-[4-oxo-5-carboxamido-9-[3-fluoro-5-(trifluoromethyl)benzyl]-*9H*-pyrido[3,4-*b*]indolyl]acetic acid;

2-[4-oxo-5-carboxamido-9-[4-fluoro-2-(trifluoromethyl)benzyl]-*9H*-pyrido[3,4-*b*]indolyl]acetic acid;

2-[4-oxo-5-carboxamido-9-[4-fluoro-3-(trifluoromethyl)benzyl]-*9H*-pyrido[3,4-*b*]indolyl]acetic acid;

2-[4-oxo-5-carboxamido-9-[2-fluoro-6-(trifluoromethyl)benzyl]-*9H*-pyrido[3,4-*b*]indolyl]acetic acid;

2-[4-oxo-5-carboxamido-9-(2,3,6-trifluorobenzyl)-*9H*-pyrido[3,4-*b*]indolyl]acetic acid;

2-[4-oxo-5-carboxamido-9-(2,3,5-trifluorobenzyl)-*9H*-pyrido[3,4-*b*]indolyl]acetic acid;

2-[4-oxo-5-carboxamido-9-(2,4,5-trifluorobenzyl)-*9H*-pyrido[3,4-*b*]indolyl]acetic acid;

2-[4-oxo-5-carboxamido-9-(2,4,6-trifluorobenzyl)-*9H*-pyrido[3,4-*b*]indolyl]acetic acid;

2-[4-oxo-5-carboxamido-9-(2,3,4-trifluorobenzyl)-*9H*-pyrido[3,4-*b*]indolyl]acetic acid;

2-[4-oxo-5-carboxamido-9-(3,4,5-trifluorobenzyl)-*9H*-pyrido[3,4-*b*]indolyl]acetic acid;

2-[4-oxo-5-carboxamido-9-[3-(trifluoromethoxyl)benzyl]-*9H*-pyrido[3,4-*b*]indolyl]acetic acid;

2-[4-oxo-5-carboxamido-9-[4-(trifluoromethoxyl)benzyl]-9*H*-pyrido[3,4-*b*]indolyl]acetic acid;

2-[4-oxo-5-carboxamido-9-[4-methoxy(tetrafluoro)benzyl]-9*H*-pyrido[3,4-*b*]indolyl]acetic acid;

2-[4-oxo-5-carboxamido-9-(2-methoxybenzyl)-*9H*-pyrido[3,4-*b*]indolyl]acetic acid;

2-[4-oxo-5-carboxamido-9-(3-methoxybenzyl)-9H-pyrido[3,4-*b*]indolyl]acetic acid;

2-[4-oxo-5-carboxamido-9-(4-methoxybenzyl)-*9H*-pyrido[3,4-*b*]indolyl]acetic acid;

2-[4-oxo-5-carboxamido-9-(4-ethylbenzyl)-9H-pyrido[3,4-b]indolyl]acetic acid;

2-[4-oxo-5-carboxamido-9-(4-isopropylbenzyl)-*9H*-pyrido[3,4-*b*]indolyl]acetic acid;

2-[4-oxo-5-carboxamido-9-(3,4,5-trimethoxybenzyl)-*9H*-pyrido[3,4-*b*]indolyl]acetic acid;

2-[4-oxo-5-carboxamido-9-(3,4-methylenedioxybenzyl)-*9H*-pyrido[3,4-*b*]indolyl]acetic acid;

2-[4-oxo-5-carboxamido-9-(4-methoxy-3-methylbenzyl)-*9H*-pyrido[3,4-*b*]indolyl]acetic acid;

2-[4-oxo-5-carboxamido-9-(3,5-dimethoxybenzyl)-*9H*-pyrido[3,4-*b*]indolyl]acetic acid;

2-[4-oxo-5-carboxamido-9-(2,5-dimethoxybenzyl)-*9H*-pyrido[3,4-*b*]indolyl]acetic acid;

2-[4-oxo-5-carboxamido-9-(4-ethoxybenzyl)-*9H*-pyrido[3,4-*b*]indolyl]acetic acid;

2-[4-oxo-5-carboxamido-9-(cyclohexylmethyl)-*9H*-pyrido[3,4-*b*]indolyl]acetic acid;

2-[4-oxo-5-carboxamido-9-(cyclopentylmethyl)-*9H*-pyrido[3,4-*b*]indolyl]acetic acid;

2-[4-oxo-5-carboxamido-9-ethyl-*9H*-pyrido[3,4-*b*]indolyl]acetic acid;

2-[4-oxo-5-carboxamido-9-(1-propyl)-*9H*-pyrido[3,4-*b*]indolyl]acetic acid;

2-[4-oxo-5-carboxamido-9-(2-propyl)-*9H*-pyrido[3,4-*b*]indolyl]acetic acid;

2-[4-oxo-5-carboxamido-9-(1-butyl)-*9H*-pyrido[3,4-*b*]indolyl]acetic acid;

2-[4-oxo-5-carboxamido-9-(2-butyl)-*9H*-pyrido[3,4-*b*]indolyl]acetic acid;

2-[4-oxo-5-carboxamido-9-isobutyl-*9H*-pyrido[3,4-*b*]indolyl]acetic acid;

2-[4-oxo-5-carboxamido-9-[2-(1-phenylethyl)]-*9H*-pyrido[3,4-*b*]indolyl]acetic acid;

2-[4-oxo-5-carboxamido-9-[3-(1-phenylpropyl)]-*9H*-pyrido[3,4-*b*]indolyl]acetic acid;

2-[4-oxo-5-carboxamido-9-[4-(1-phenylbutyl)]-9*H*-pyrido[3,4-*b*]indolyl]acetic acid;

2-[4-oxo-5-carboxamido-9-(1-pentyl)-*9H*-pyrido[3,4-*b*]indolyl]acetic acid; and

2-[4-oxo-5-carboxamido-9-(1-hexyl)-*9H*-pyrido[3,4-*b*]indolyl]acetic acid;

or a pharmaceutically acceptable racemate, solvate, tautomer, optical isomer, prodrug derivative or salt thereof.

**Synthesis Methods**

Compounds of formula I where the A ring is phenyl and the heteroatom in Z is sulfur, oxygen or nitrogen can be prepared as described in Schemes I(a) - (f), below.

Scheme I(a)

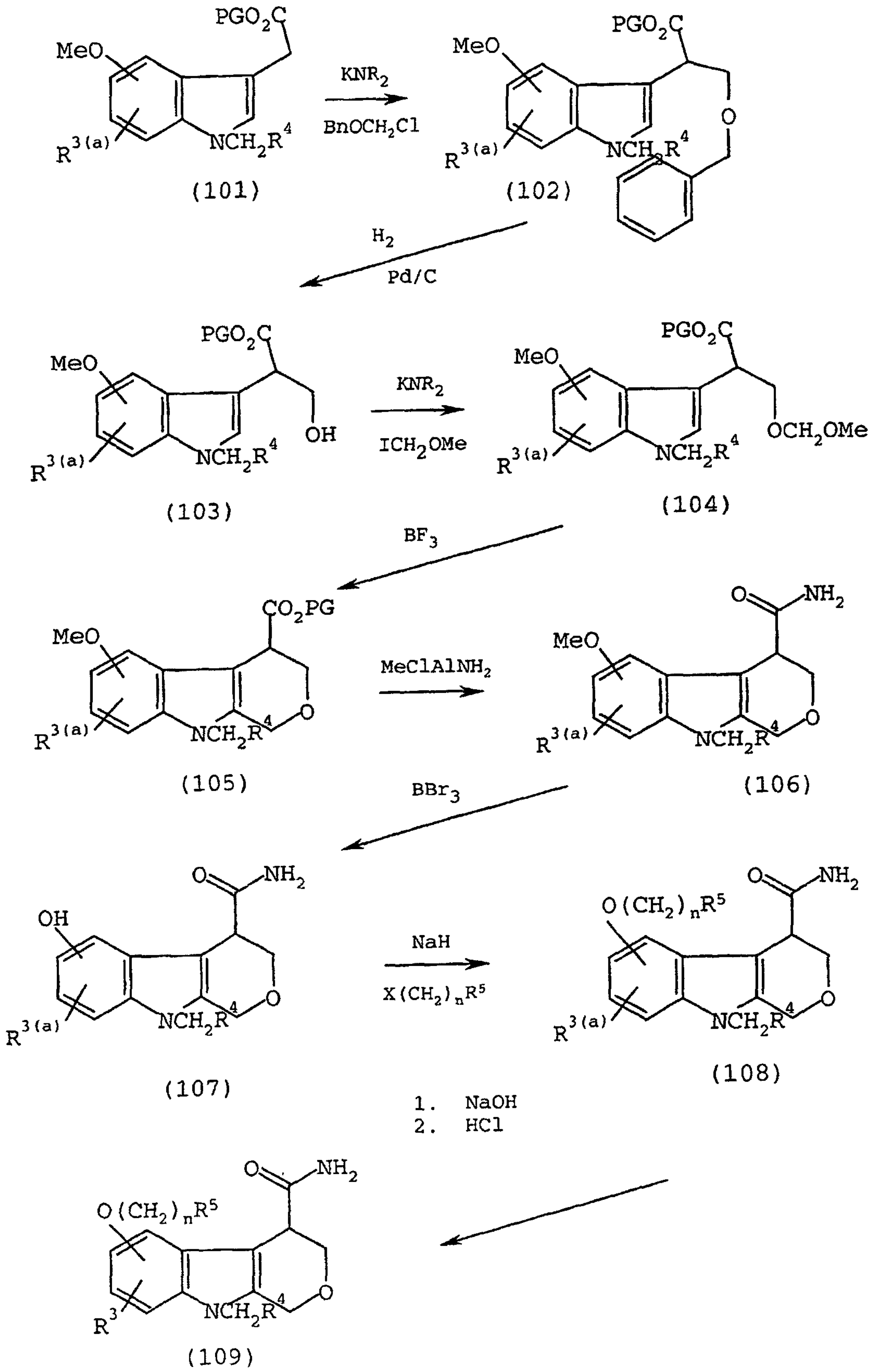


(101) (102) (103) (104) (105) (106) (107) (108) (109)

PG is an acid protecting group.

X is halo.

$R^3$(a) is H, -O($C_1$-$C_4$)alkyl, halo, -($C_1$-$C_6$)alkyl, phenyl, - ($C_1$-$C_4$)alkylphenyl; phenyl substituted with -($C_1$-$C_6$)alkyl, halo or -$CF^3$; -$CH_2OSi$($C_1$-$C_6$)alkyl, furyl, thiophenyl, -($C_1$-$C_6$)hydroxyalkyl; or -$(CH_2)_nR^8$ where $R^8$ is H, -$NR^9R^{10}$, -CN or phenyl where $R^9$ and $R^{10}$ are independently -($C_1$-$C_4$)alkyl or -phenyl($C_1$-$C_4$)alkyl and n is 1 to 8;

An indole-3-acetic ester (101), Ref 10, is alkylated by treatment with alkalai metal amide and benzyloxymethyl chloride to give (102) which is converted to the alcohol (103) by catalytic hydrogenation. The alcohol is alkylated to provide the formaldehyde acetal (104) which is cyclized by Lewis acid to produce the pyrano[3,4-b]indole (105). The ester is converted to the amide (106) by methylchloroaluminum amide, and then to the phenol (107) with boron tribromide. The phenol is O-alkylated to give (108) which is hydrolyzed to the acid (109).

10) Dillard, R. et al., J, Med Chem. Vol 39, No. 26, 5119-5136.

**Scheme I(b)**

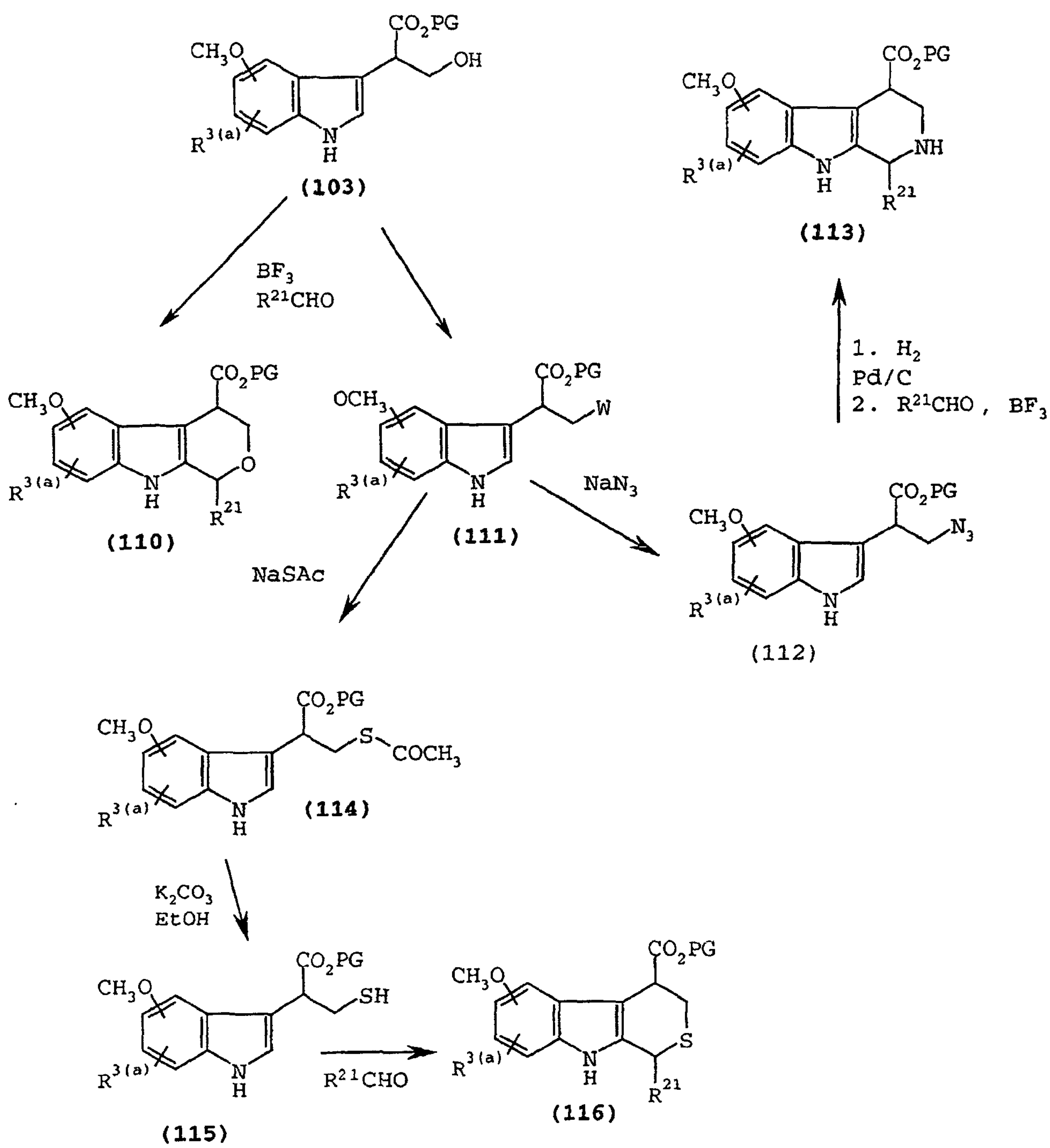


(103) (110) (111) (112) (113) (114) (115) (116)

PG is an acid protecting group

W is halo, alkyl or aryl sulfonyl

$R^3$(a) is H, -O($C_1$-$C_4$)alkyl, halo, -($C_1$-$C_6$)alkyl, phenyl, - ($C_1$-$C_4$)alkylphenyl; phenyl substituted with -($C_1$-$C_6$)alkyl, halo or -$CF^3$; -$CH_2OSi$($C_1$-$C_6$)alkyl, furyl, thiophenyl, -($C_1$-$C_6$)hydroxyalkyl; or -$(CH_2)_nR^8$ where $R^8$ is H, -$NR^9R^{10}$, -CN or phenyl where $R^9$ and $R^{10}$ are independently -($C_1$-$C_4$)alkyl or -phenyl($C_1$-$C_4$)alkyl and n is 1 to 8;

Reaction of this alcohol (103) with aldehyde and acid produces the pyranoindole (110).

Conversion of the hydroxyl function of (103) to a halide or sulfate functionality is achieved by treatment with triphenylphosphine and $CH_3X$ (where X is a halogen) to make compounds of formula (111) where X is a halide; or by treatment with triethylamine and methanesulfonyl chloride to make the sulfonate. Displacement with the sodium salt of thiol acetic acid gives (114) which in turn is hydrolyzed by base to the thiol (115) which is reacted with an appropriately substituted aldehyde and acid to produce the thiopyranoindoles (116).

Intermediate (111) may also be reacted with sodium azide to give the azido derivative (112) which is reduced by hydrogen catalytically to give the amine which is converted to the carboline (113) with aldehyde and acid.

Intermediates (113), (110) and (116) may be N-alkylated, using sodium hydride and an appropriately substituted alkylhalide $XCH_2R^4$.

Scheme I(c)

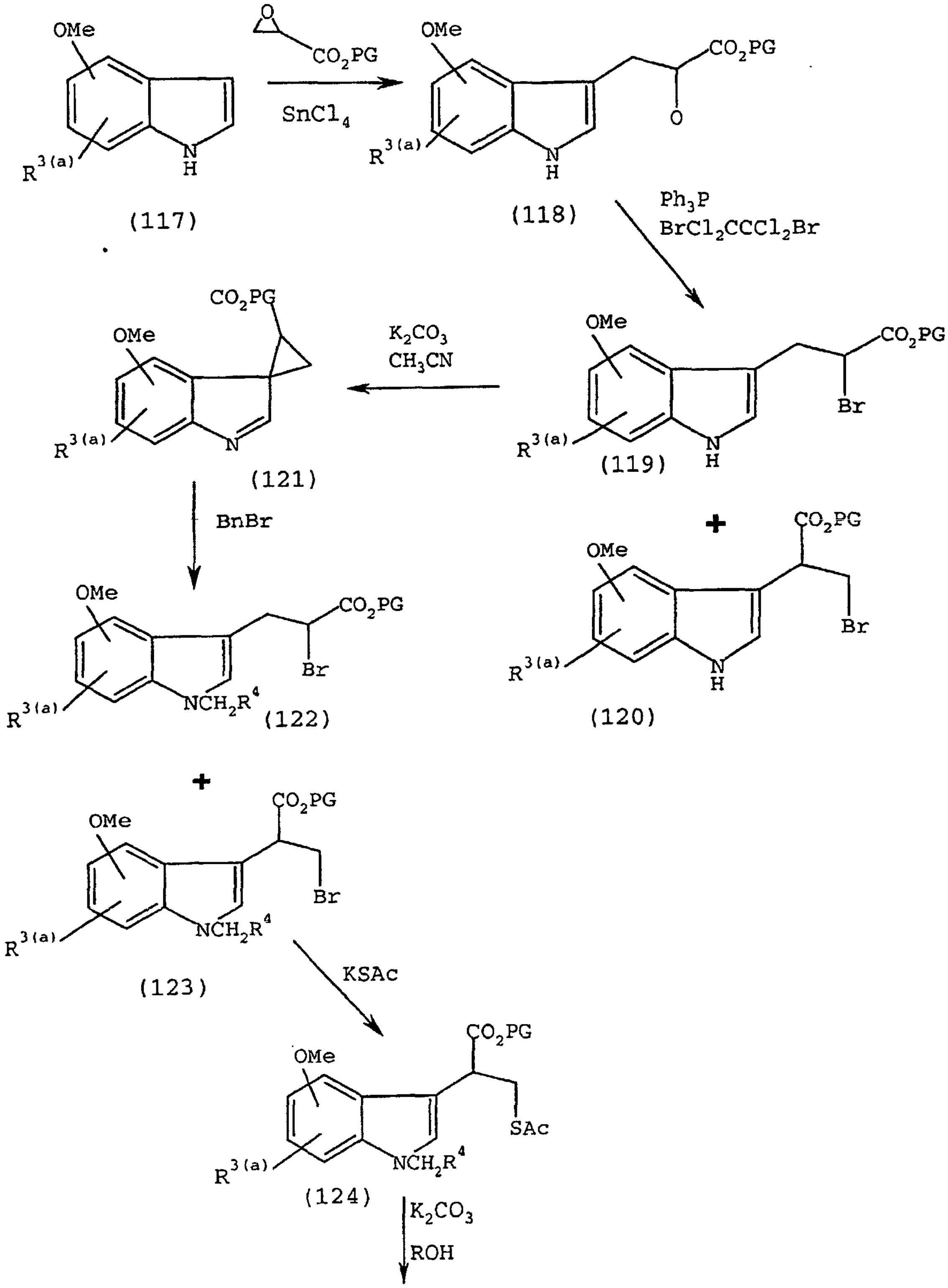


(117) (118) (119) (120) (121) (122) (123) (124)

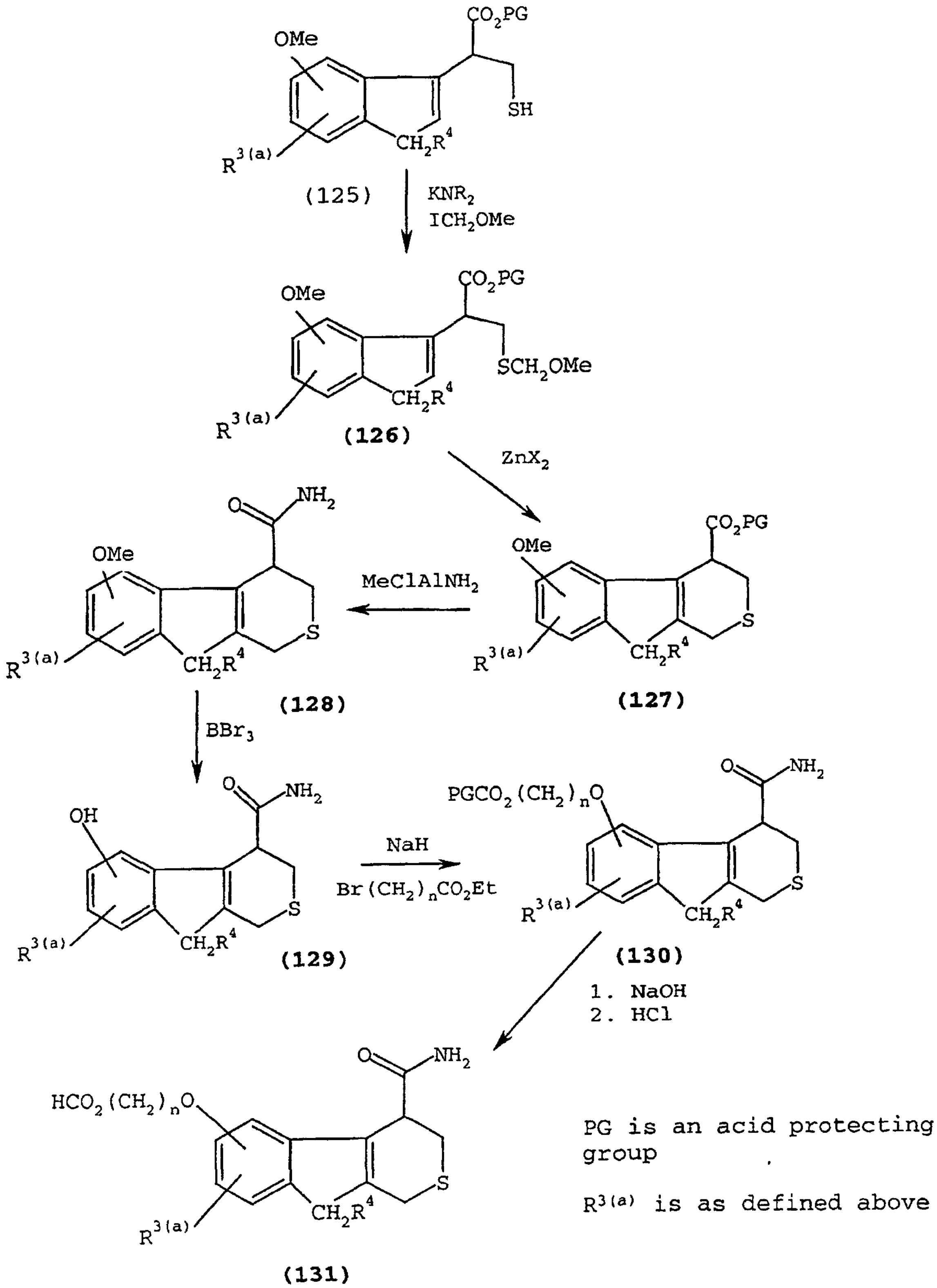


4-Methoxyindole (117) is converted to the indol acetic acid derivative (118) by alkylation with an epoxy propionate. Treatment of (118) with a brominating reagent affords the mixture of bromo isomers (119) and (120) which give the spiro compound (121) upon basic treatment. Heating (121) with benzyl bromide provides a mixture of the

isomeric bromo compounds (122) and (123) which react with potassium thioacetate to give a mixture of isomers from which (124) may be separated. Solvolysis of the thioester produces the thiol (125) which is alkylated to give (126). Lewis acids convert (126) to the thiopyrano[3,4-b]indole (127). The ester function is converted to amide using methylchloroaluminum amide, the methyl ether cleaved by boron tribromide, and the product phenol O-alkylated with bromoacetic ester to give (130) which is hydrolyzed to (131).

## Scheme I(d)

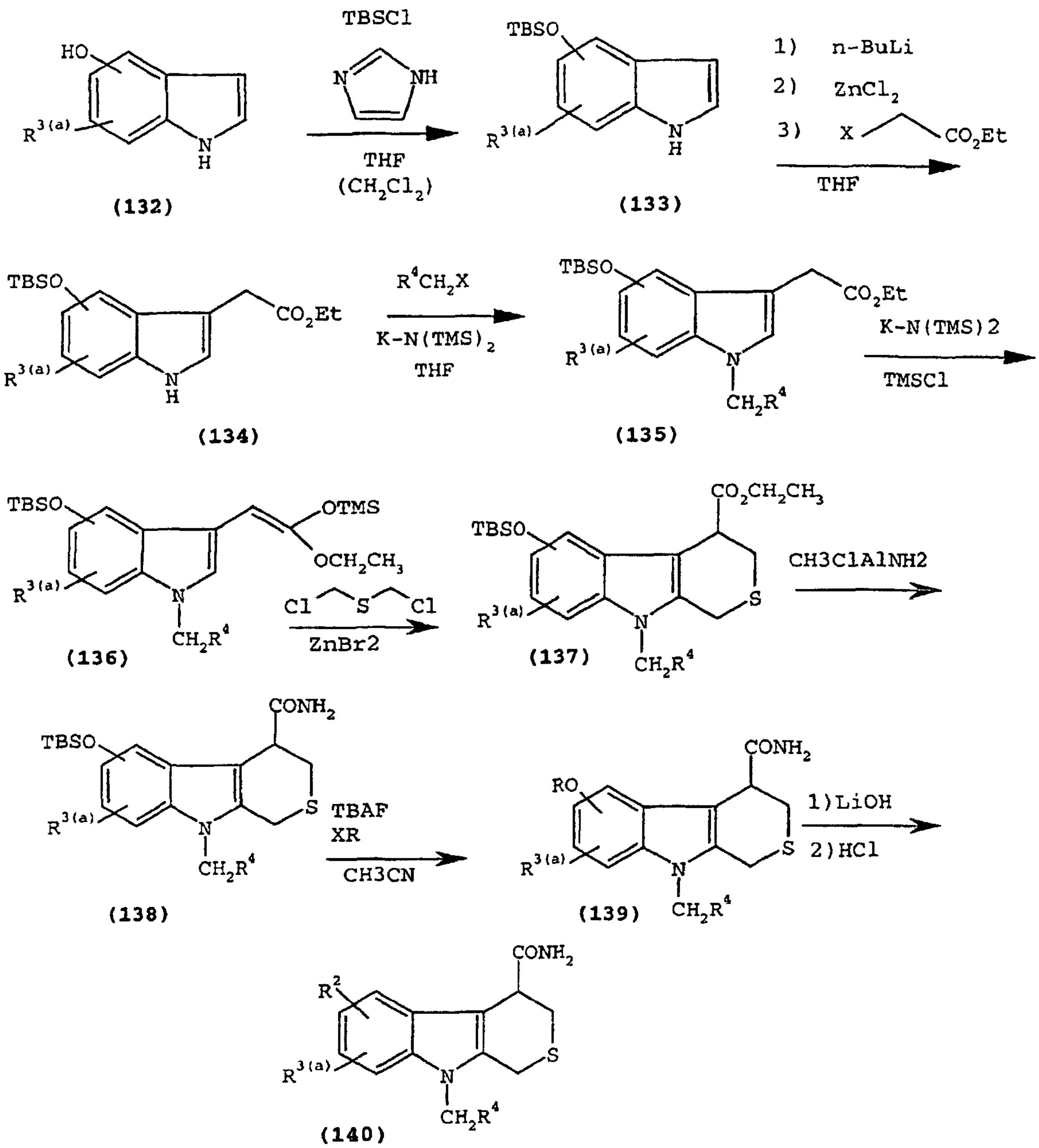


(132) (133) (134) (135) (136) (137) (138) (139) (140)

X is halo,

$R^{3(a)}$ is as defined in Scheme I(a) above; and

R is - $(CH_2)mR^5$.

Protection of the oxygen by treatment of (132) with *tert*-butyldimethylsilyl chloride and imidazole in an aprotic polar solvent such as tetrahydrofuran or methylene chloride accomplishes (133).

Alkylation at the 3-position of the indole (133) is achieved by treatment with n-butyllithium then zinc chloride at temperatures starting at about 10°C and warming to room temperature, followed by reaction with an appropriate

haloalkyl ester such as methyl or ethyl bromoacetate. The reaction is preferably conducted at room temperature in an appropriate aprotic polar solvent such as tetrahydrofuran.

Alkylation of the indole-nitrogen can then be achieved by reacting (134) with a suitable alkyl halide in the presence of potassium bis(trimethylsilyl)amide to prepare (135).

The ester functionality of (135) is converted to a trimethylsilylketene acetal (136) by treatment with potassium bis(trimethylsilyl)amide and trimethylsilyl chloride. Treatment of the ketene acetal (136) with bis(chloromethyl)sulfide and zinc bromide in methylene chloride affords the cyclized product (137). Conversion to amide (138) can be accomplished by a Weinreb reaction with methylchloroaluminum amide. Removal of the oxygen protecting group with a fluoride source, such as tetrabutylammonium fluoride (TBAF), and concommitant reaction of the resulting anion with, for example, ethyl bromoacetate yields the ester (139). Deprotection of the ester yields the desired acid (140).

Scheme I(e)

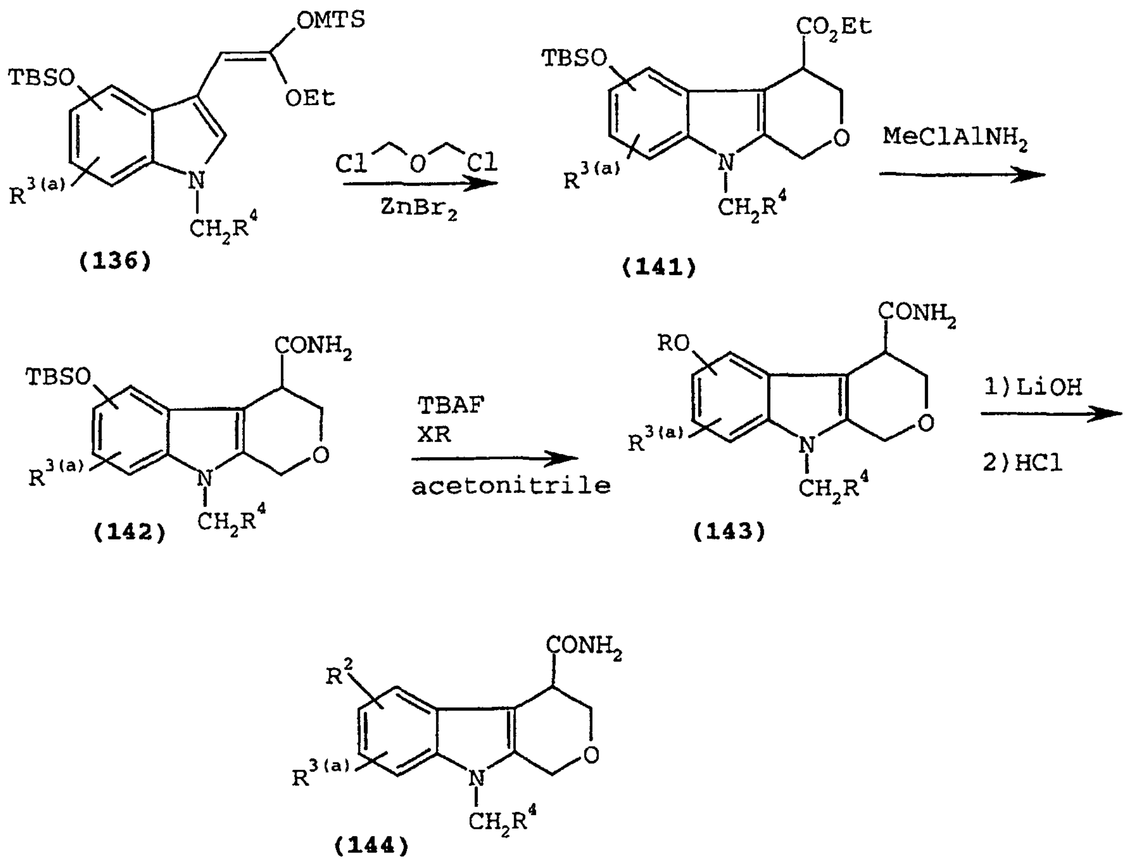


(136) (141) (142) (143) (144)

$R^{3(a)}$ is as described in Scheme I(a) and
R is as described in Scheme III(b).

Treatment of the ketene acetal (136) with bis(chloromethyl)ether and zinc bromide in methylene chloride affords the cyclized product (141). Conversion to amide (142) can be accomplished by a Weinreb reaction with methylchloroaluminum amide. Removal of the oxygen protecting group with a fluoride source, such as tetrabutylammonium fluoride, and concommitant reaction of the resulting anion with ethyl bromoacetate yields the ester (143). Deprotection of the ester yields the desired acid (144).

Scheme I(f)

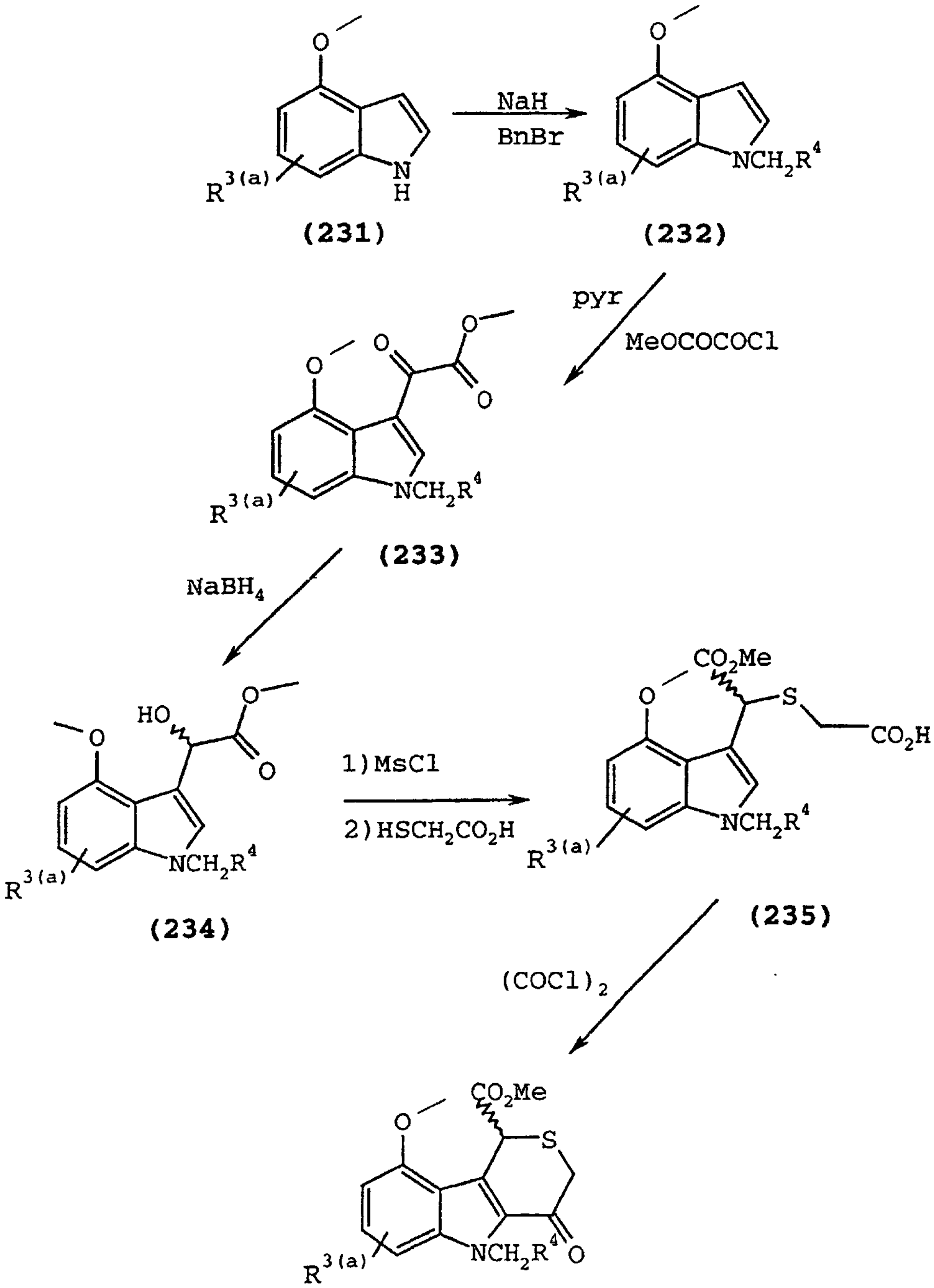


(231) (232) (233) (234) (235) (236)

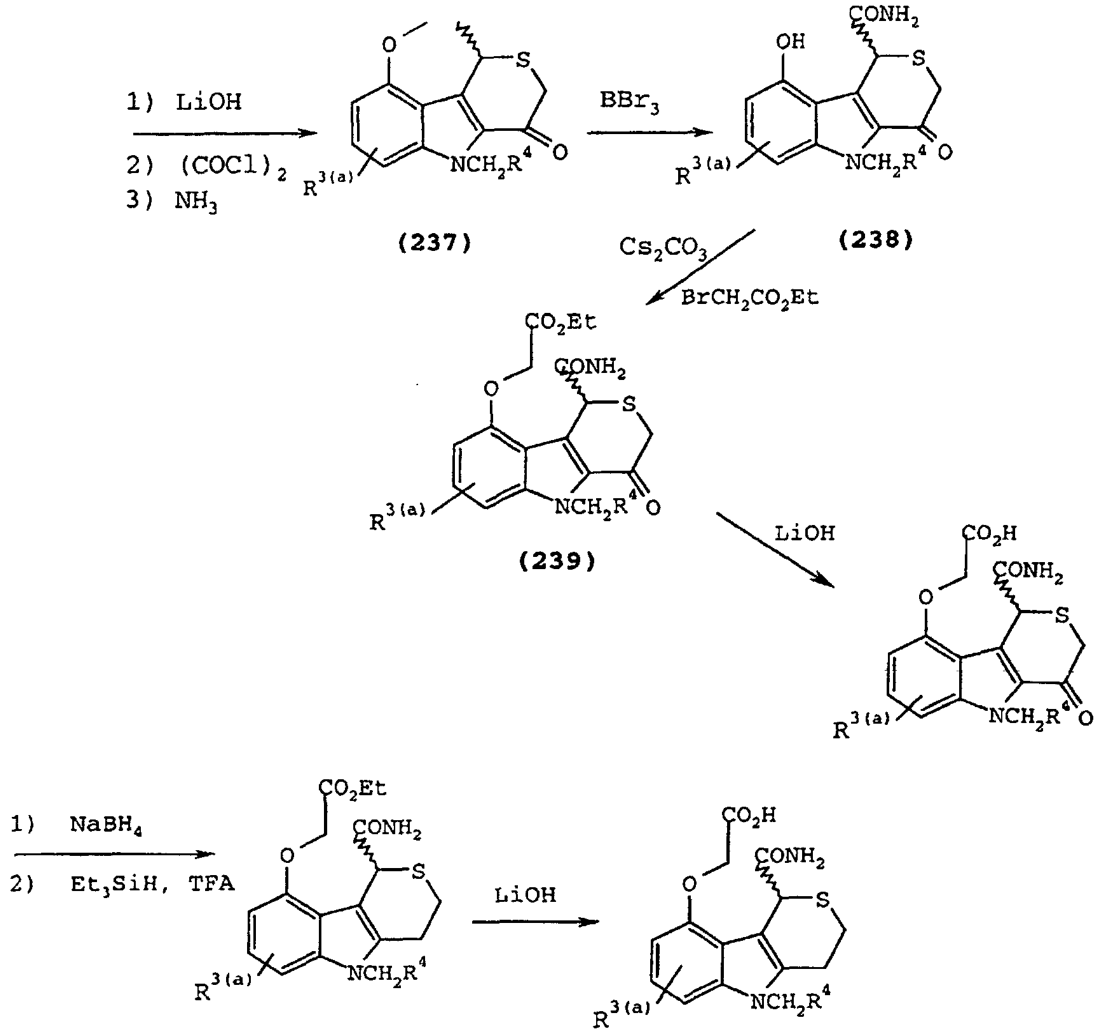


(237) (238) (239) (241) (242)

N-alkylation of commercially available 4-methoxy indole (231) under basic conditions using an alkyl halide affords the N-alkyl indole (232). Acylation with a suitable acid chloride provides the glyoxalate ester product (233) which can be reduced with a variety of hydride reducing agents to give intermediate alcohols (234). Conversion of the alcohol to a suitable leaving group and displacement with sulfur nucleophiles affords the thioether product (235). Conversion to the acid chloride and spontaneous cyclization affords the thioketone product (236). Cleavage of the ester can be effected under basic conditions to give the correponding acid which upon formation of the acid chloride and reaction with an appropriate amine gives the amide product (237). Cleavage of the methyl ether gives the phenol (238) which can be alkylated under basic conditions using alkyl halides to give the O-alkylated product (239). Cleavage of the ester under basic conditions gives the desired product (240). Alternatively, reduction of the benzylic ketone with a hydride reducing agent and subsequent deoxygenation of the resulting alcohol gives the deoxygenated product (244). Cleavage of the oxyacetic ester proceeds under basic conditions to give the desired oxyacetic acid (242).

Compounds where Z is an aromatic or heterocyclic ring containing nitrogen can be prepared as described in Schemes II(a)-(e), below.

Scheme II(a)

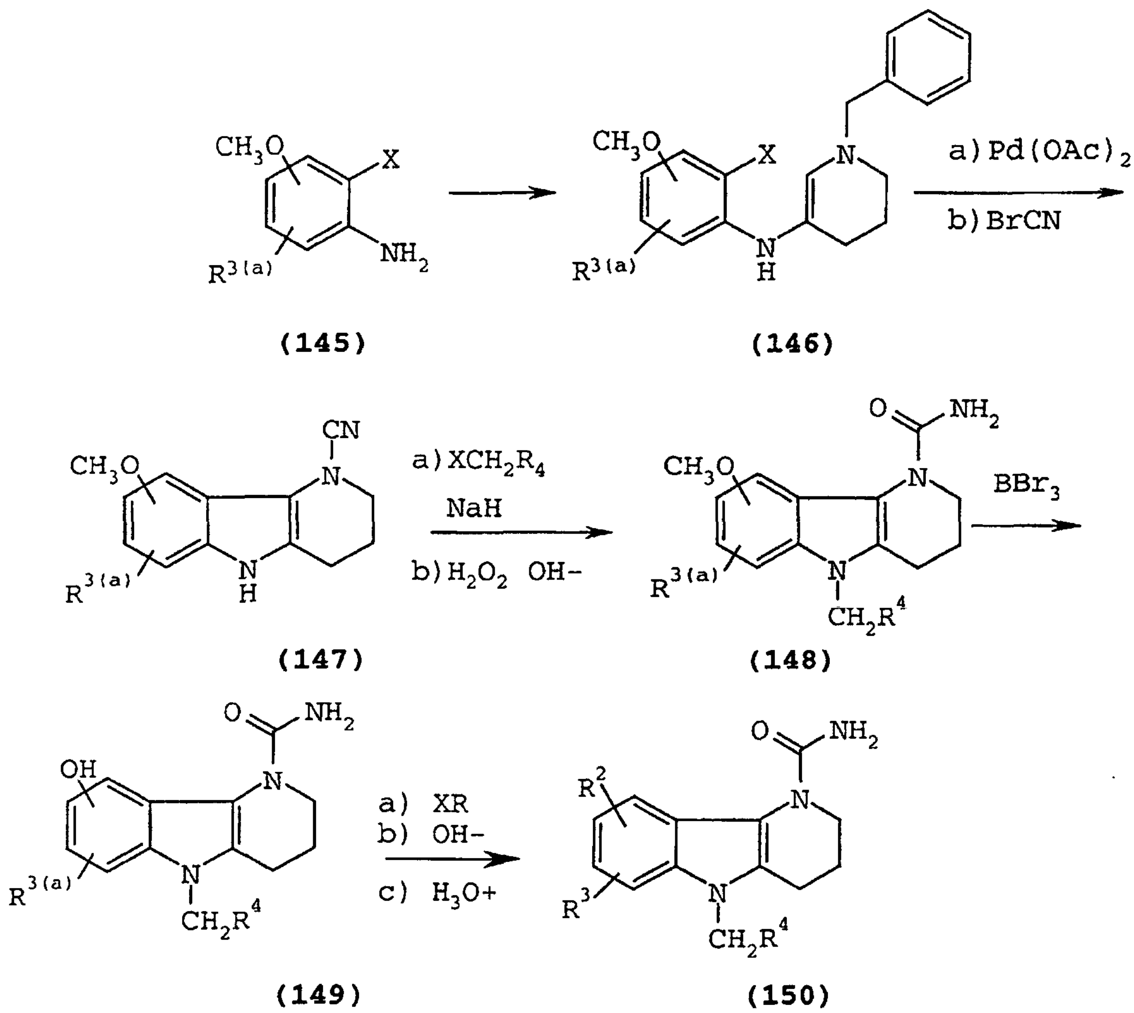


(145) (146) (147) (148) (149) (150)

Substituted haloaniline (145) is condensed with N-benzyl-3-piperidone to provide enamine (146). Ring closure is effected by treatment of (146) with palladium (II) acetate and the resultant product is converted to (147) by treatment with cyanogen bromide. Alkylation of (147) is accomplished by treatment with the appropriate alkyl bromide using sodium hydride as base. Hydrolysis of this N-alkylated product with basic hydrogen peroxide under standard conditions provides (148). Demethylation of (148) is carried out by treatment with boron tribromide in methylene chloride. The resulting phenol (149) is converted by the standard sequence of O-alkylation with methyl bromoacetate in the presence of a base, hydrolysis with hydroxide to provide the intermediate salt which is then protonated in aqueous acid to provide desired δ-carboline (150).

Scheme II(b)

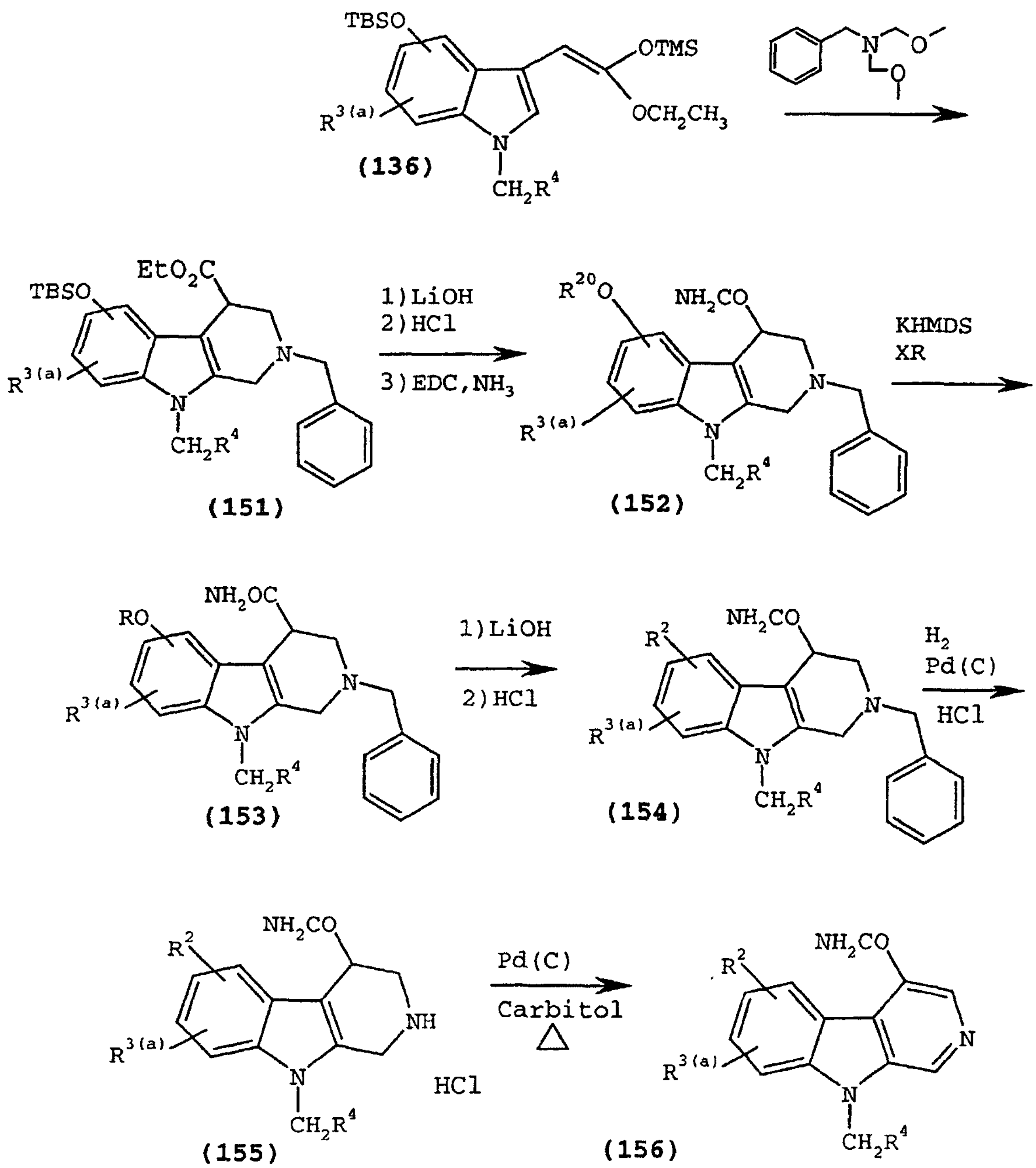


(136) (151) (152) (153) (154) (155) (156)

| | |
|---|---|
| X | is halo, |
| R | is as defined in Scheme I(d), and |
| $R^{3(a)}$ | is as defined in Scheme I(a). |

Ketene acetal (136), prepared as described in Scheme I(d), is reacted with benzyl bis(methoxymethyl)amine in the presence of zinc chloride to give the tetrahydro-beta-carboline (151).

Treatment of (151) with lithium hydroxide, neutralization with hydrochloric acid and subsequent treatment with 1-(3-dimethylaminopropyl)-3-ethylcarbodiimide hydrochloride and ammonia provides the desilyated amide (152) where $R^{20}$ is hydrogen, which can be alkylated with, for example, ethylbromoacetate to give ester (153).

Alternatively, treatment of (115) with the appropriate Weinreb reagent provides amide (152) ($R^{20}$ is t-butyldimethylsilyl) which is desilylated with tetra-n-butylammonium fluoride and alkylated with, for example, ethyl bromoacetate to give ester (153). Lithium hydroxide-mediated hydrolysis gives acid (154), which may be hydrogenated over an appropriate catalyst in the presence of hydrochloride acid to give the tetrahydro-beta-carboline as the hydrochloride salt(155). Compound (155) may in turn be aromatized by refluxing in carbitol with palladium on carbon to provide beta-carboline (156).

Scheme II(c)

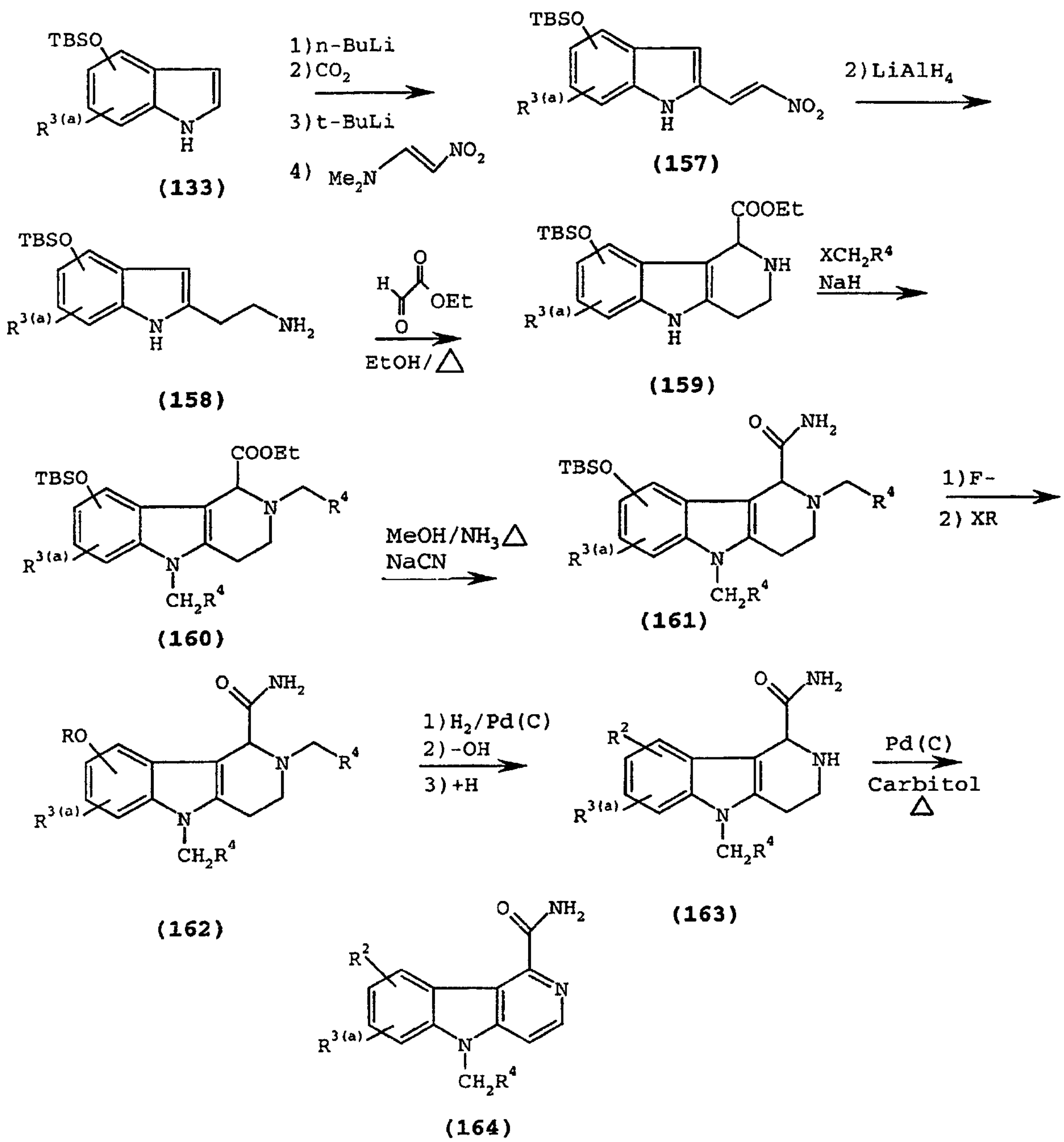


| | |
|---|---|
| X | is halo, |
| R | is as defined in Scheme I(d); and |
| $R^{3(a)}$ | is as defined in Scheme I(a). |

In a one-pot reaction, indole (133) is successively treated with one equivalent n-butyllithium, carbon dioxide gas, one equivalent of t-butyllithium, and 1-dimethylamino-2-nitroethene to give (157). Nitroalkene (157) is reduced with lithium aluminum hydride to amine (158), which is cyclized with methyl glyoxylate (Ref. 9) in refluxing ethanol to

give tetrahydrocarboline (159). Alkylation of both nitrogens of (159) leads to intermediate (160), which is treated with the appropriate Weinreb reagent to provide amide (161). Fluoride-assisted desilylation and alkylation with, for example, ethyl iodoacetate gives ester (162), which may be hydrogenated over a suitable catalyst and base-hydrolyzed to give acid (163). Aromatization of (163) to carboline (164) is achieved by refluxing in carbitol in the presence of palladium-on-carbon.

Reference 9:

Kelley, T. R.; Schmidt, T. E.; Haggerty, J. G. A convenient preparation of methyl and ethyl glyoxylate, *Synthesis,* **1972,** 544-5.

Scheme II(d)

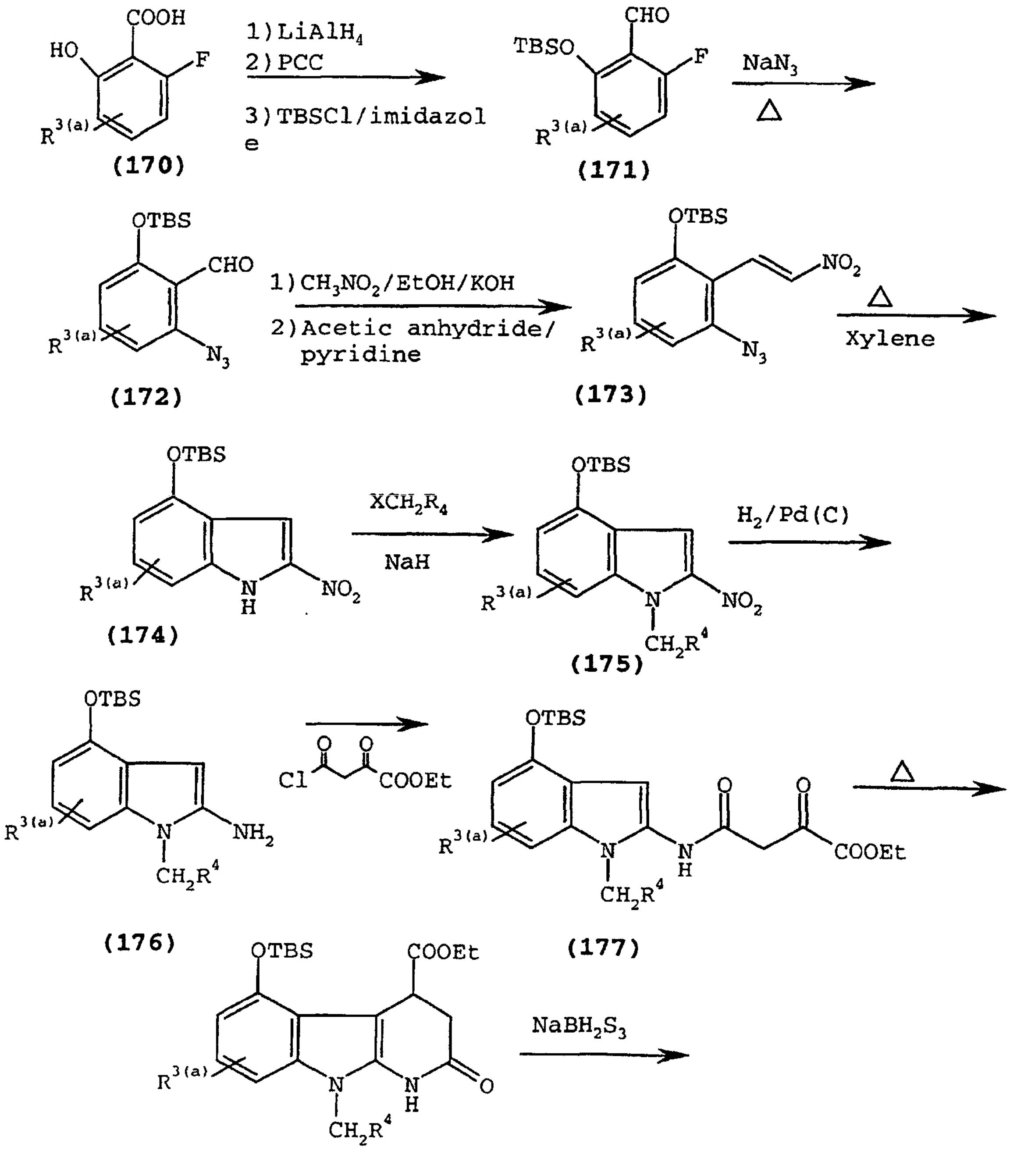


(170) (171) (172) (173) (174) (175) (176) (177) (178)

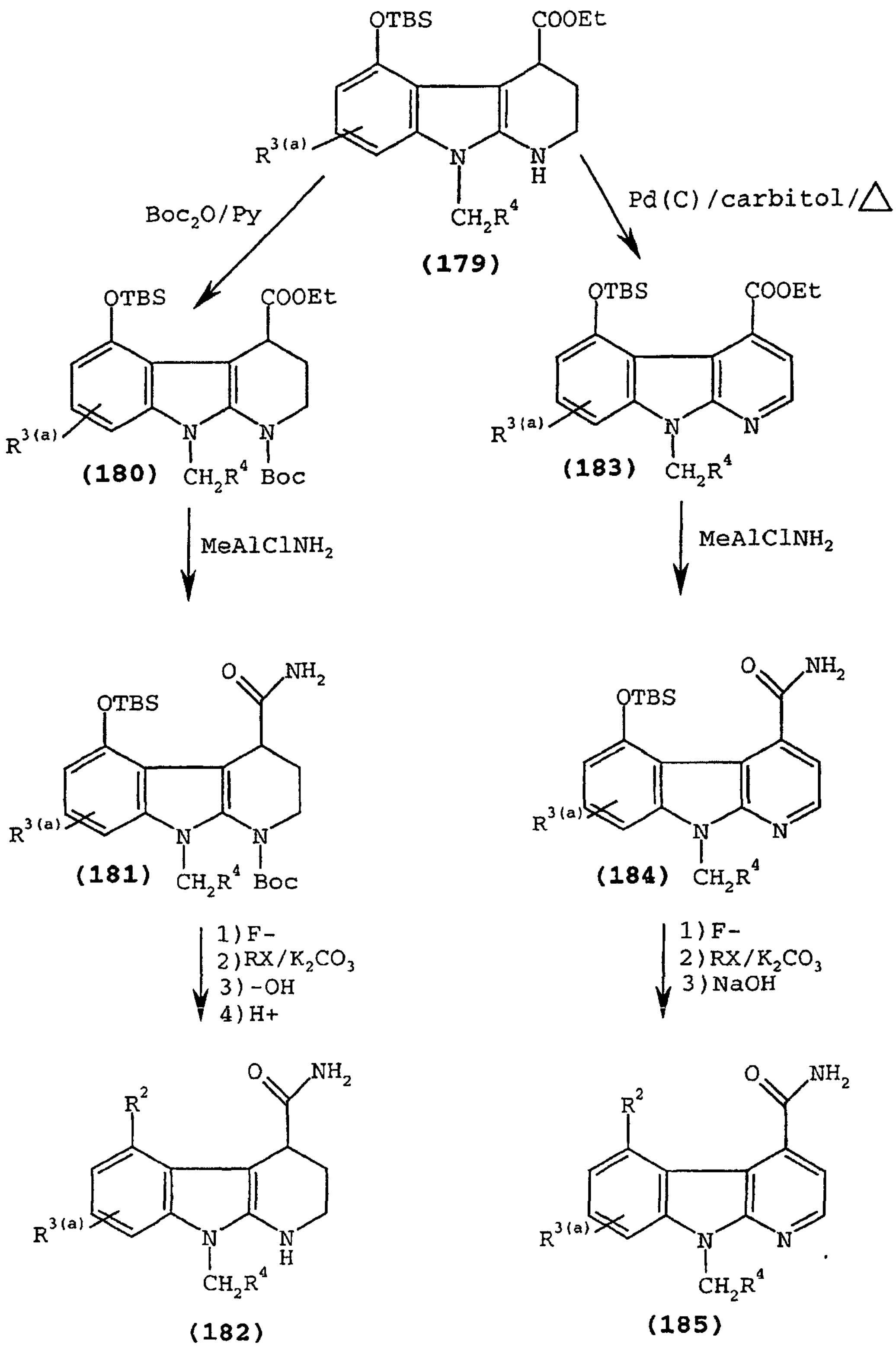


(179) (180) (183) (181) (184) (182) (185)

The commercially available acid (170) is reduced with lithium aluminum hydride, oxidized with pyridinium chlorochromate, and silylated with t-butyldimethylsilyl chloride to give (171). Treatment with sodium azide provides azide (172), which is reacted with nitromethane and potassium hydroxide in ethanol, followed by treatment with acetic anhydride and pyridine to give nitroolefin (173). Heating in xylene induces cyclization to produce indole (174). Alkylation with, for example, benzyl iodide and sodium hydride gives (175), which is hydrogenated in the presence of palladium-on-carbon

to give amine (176). Acylation with the acid chloride of commercially available oxalacetic acid monoethyl ester gives (177), which is thermally cyclized to lactam (178). Selective reduction of the lactam carbonyl may be accomplished by treatment with $NaBH_2S_3$ to provide amine (179).
Protection of amine (179) with di-t-butyl dicarbonate and pyridine produces (180), which is converted via the appropriate Weinreb reagent to amide (181). Fluoride-assisted desilylation, alkylation with, for example, ethyl iodoacetate and potassium carbonate, base hydrolysis, and acid hydrolysis produce the tetrahydro-alpha-carboline (182).

Alternatively, amine (179) may be aromatized by refluxing in carbitol or some other suitable high boiling solvent to give alpha-carboline (183), which is converted via the appropriate Weinreb reagent to amide (184). Fluoride-assisted desilylation, alkylation with ethyl iodoacetate and potassium carbonate, and base hydrolysis as described above provides alpha-carboline (185).

Scheme II(e)

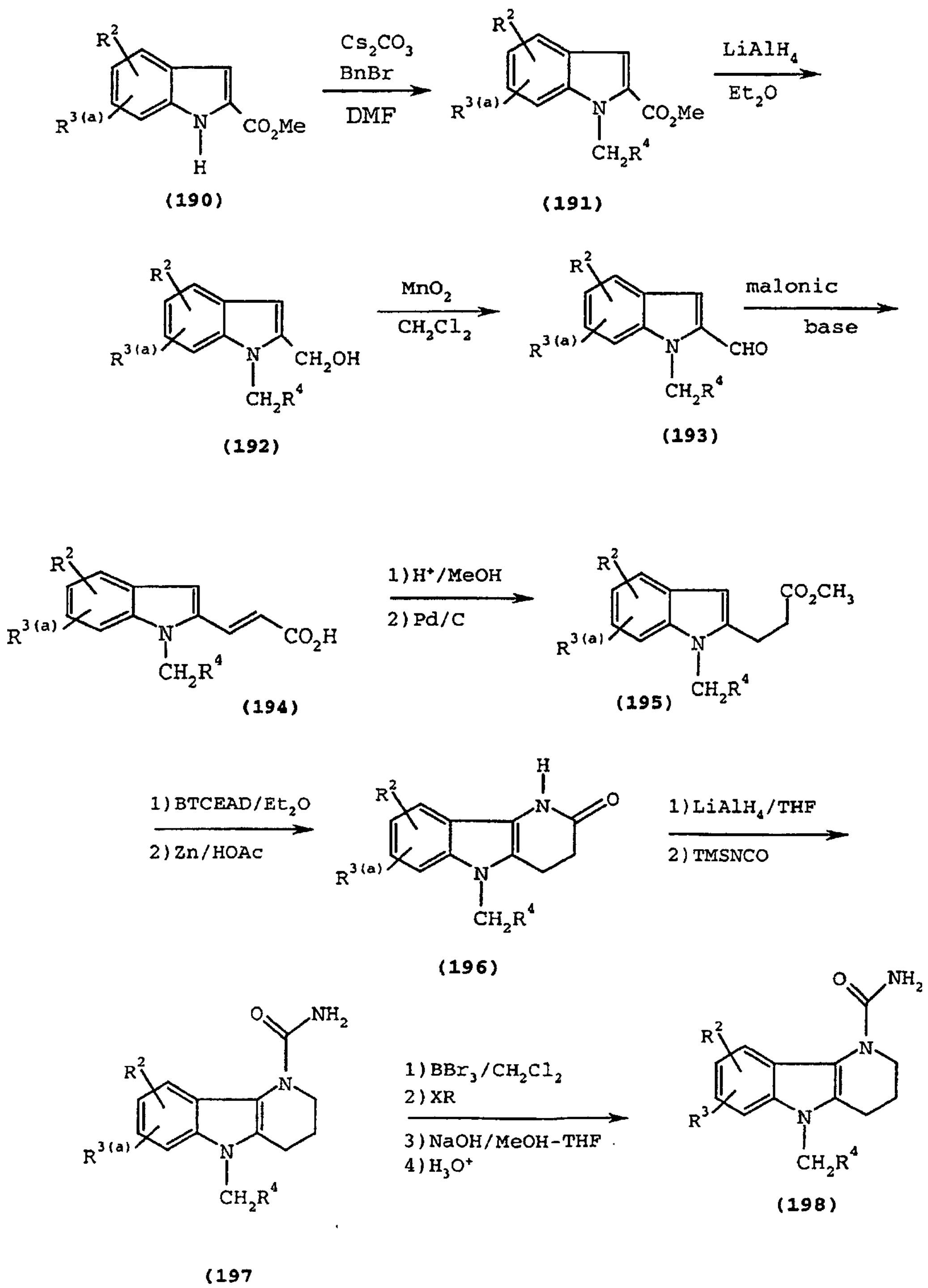


X is halo
$R^{3(a)}$ is as defined above

Scheme II(e) provides δ-carboline (198) by the indicated sequence of reactions. N-alkylation of 2-carboethoxyindole (190) followed by a standard two carbon homologation sequence provides 2-(3-propenoic acid)indoles (194). In this sequence, the condensation of aldehyde (193) with malonic acid utilized a mixture of pyridine and piperidine as the

base. After methyl ester formation and hydrogenation (195), ring closure (196) was effected by treatment with bis (2,2,2-trichloroethyl)azodicarboxylate (BTCEAD) followed by zinc in acetic acid. Reduction of the cyclic amide with lithium aluminum hydride followed by treatment with trimethylsilylisocyanate provided the urea (197).
Conversion to the desired d-carboline (198) was accomplished under the usual conditions of demethylation and subsequent alkylation and ester hydrolysis steps.

Reverse indoles, i.e., compounds where B is carbon and D is nitrogen can be prepared as described in Scheme III, below.

Scheme III

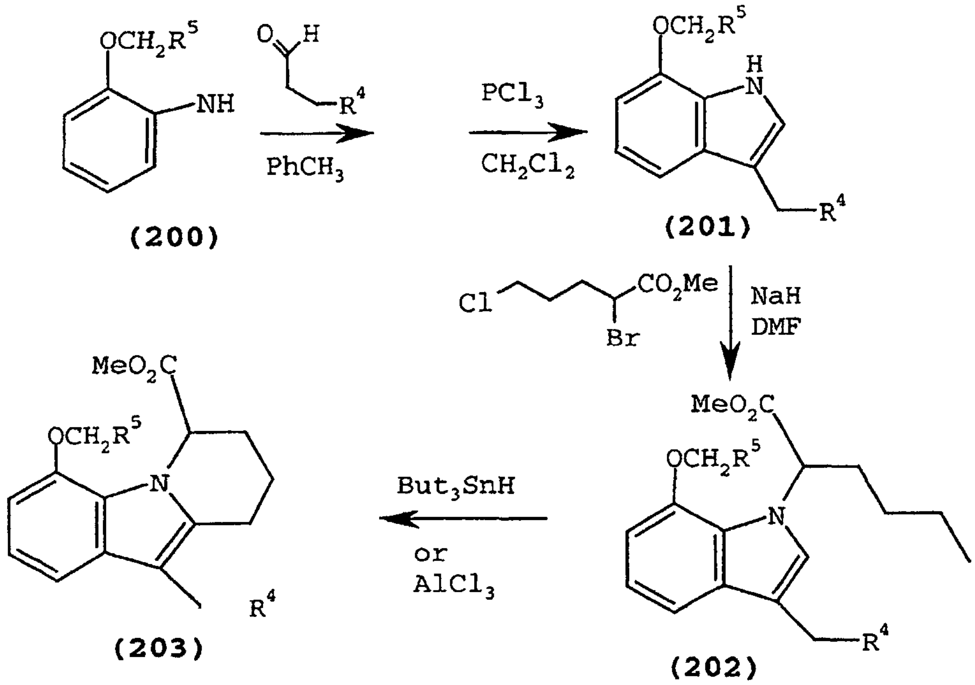


Aryl hydrazines (200) are condensed with substituted prpionaldehydes to form hydrazones which are cyclized to indoles (201) by treatment with phosphorous trichloride at room temperature (Ref 1). The indoles are N-alkylated on reaction with a base such as sodium hydride and an alph-bromo ester to give indoles (202) which are cyclized to tetrahydrocarbazoles (203) by Lewis acids (e.g., aluminum chloride) or by radical initiators (e.g., tributyltin hydride). Compounds (203) can be converted to carbazoles by, for example, refluxing in a solvent such as carbitol in the presence of Pd/C.

Compounds of formula I wherein A is pyridyl can be prepared as described in Schemes IV(a)-(b), below.

Scheme IV(a)

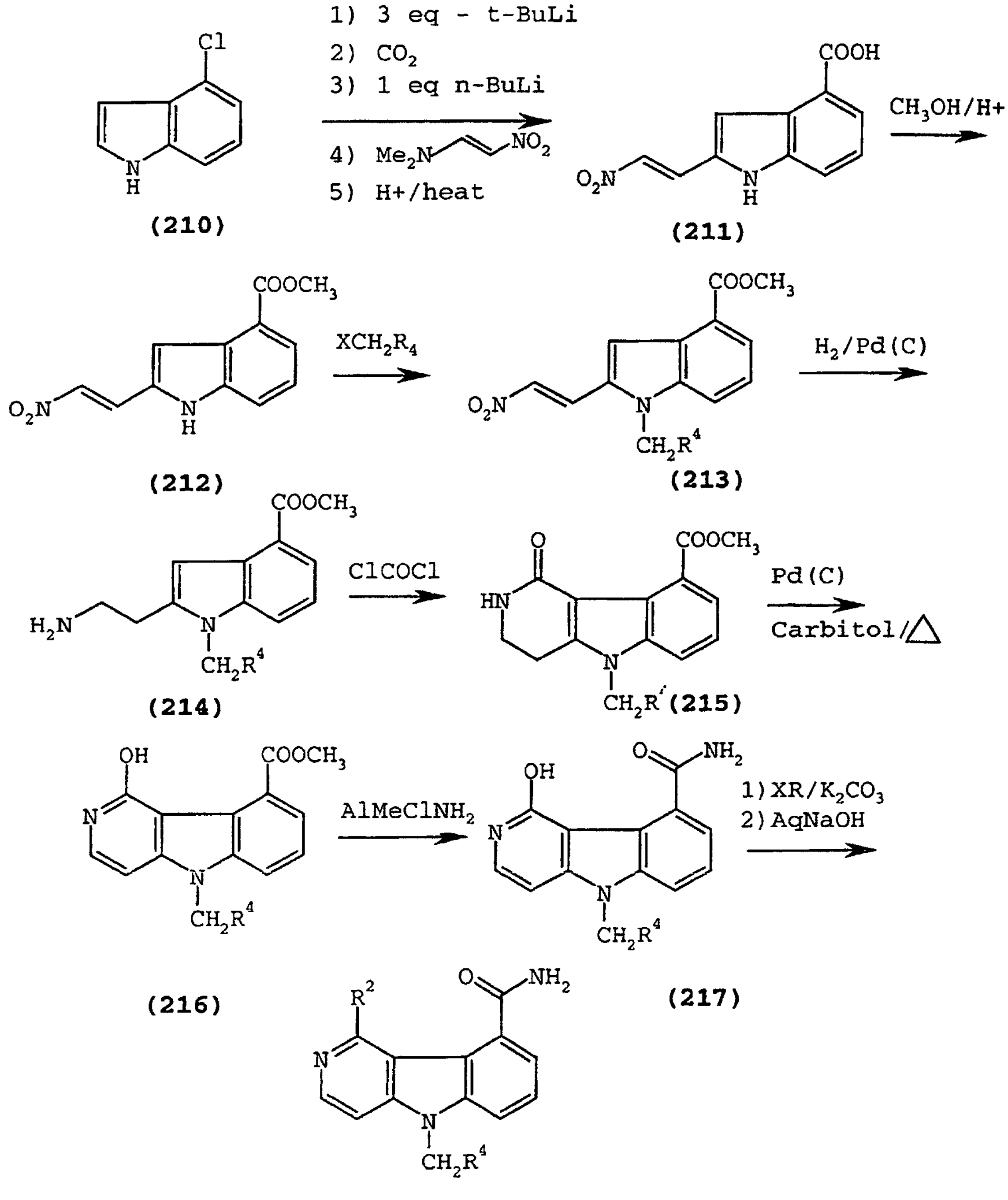


(210) (211) (212) (213) (214) (215) (216) (217) (218)

X is halo and
R is $(CH_2)_mR^5$.

Commercially available 4-chloroindole (210) is treated with 3 equivalents of t-butyllithium followed by carbon dioxide, 1 equivalent of n-butyllithium, 1-dimethylamino-2-nitroethene, and acid to provide carboxylic acid (211), which may be esterified to give (212). Alkylation at the 1-position followed by hydrogenation provides aminoethyl indole (214). Cyclization with phosgene to (215) followed by aromatization gives carboline (216). Treatment of (216) with the appropriate Weinreb reagent provides amide (217), which may be alkylated with, for example, ethyl bromoacetate and saponified with sodium hydroxide to give the carboline (218).

Scheme IV(b)

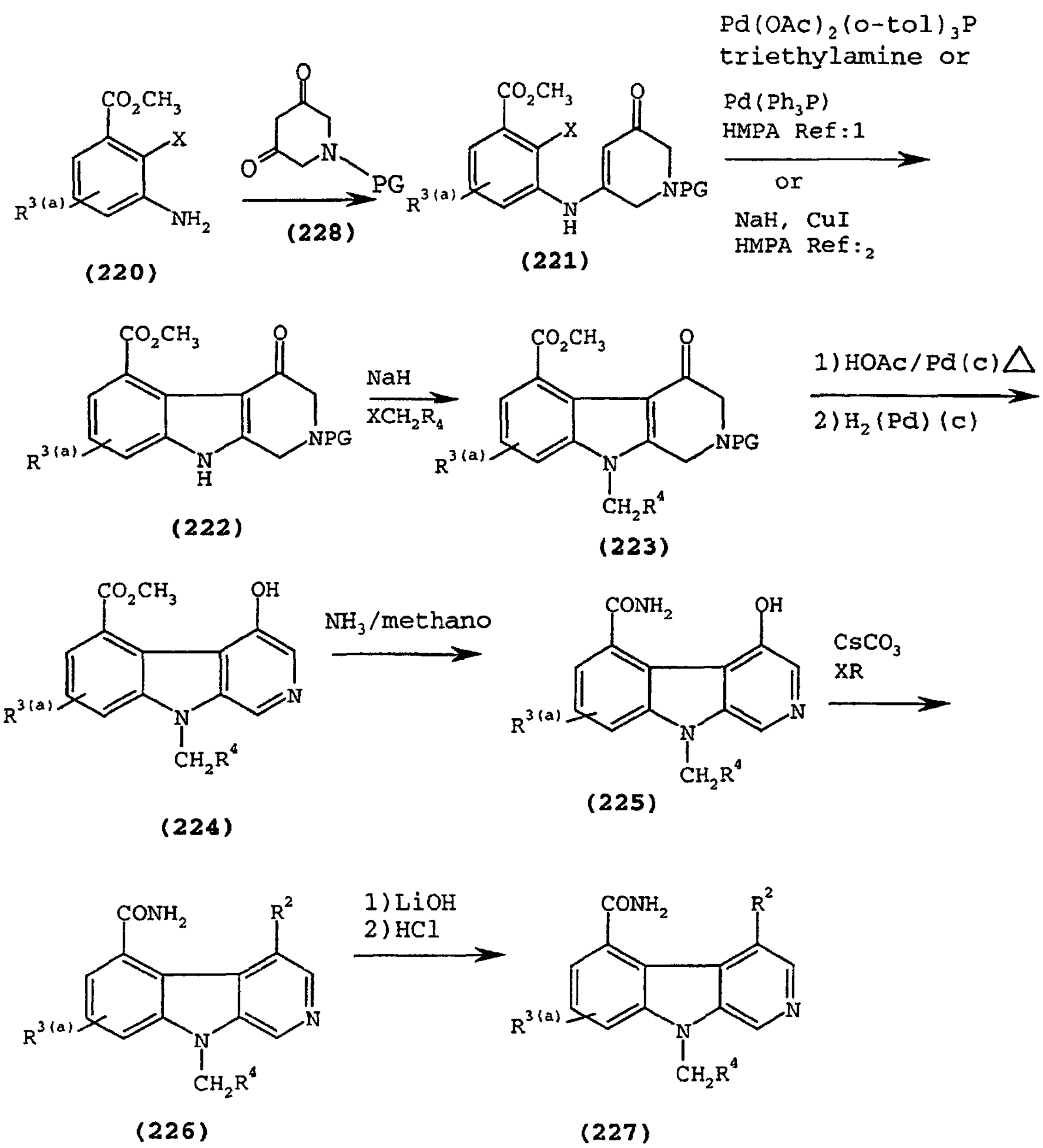


(220) (228) (221) (222) (223) (224) (225) (226) (227)

R3(a) is as defined in Scheme I(a),
X is halo, and
R is $(CH_2)mR^5$.

The 1,3-dione structures (228) are either commercially available or readily prepared by known techniques from commercially available starting materials. Preparation of the aniline derivatives (220) (X= Cl, Br, or I) are accomplished by reducing an appropriately substituted benzoic acid derivative to the corresponding aniline by treatment with a reducing agent such as $SnCl_2$ in hydrochloric acid in an inert solvent such as ethanol or by hydrogenation using hydrogen gas and sulfided platinum or carbon or palladium on carbon. The amino group of (228) is protected with an

appropriate protecting group, such as the, carboethoxyl, benzyl, CBZ (benzyloxycarbonyl) or BOC (tert-butoxycarbonyl) protecting group, and the like.

The dione (228) and aniline derivative (220) are condensed according to the general procedure of Chen, et al., (Ref 10) or Yang, et al., (Ref 11), with or without a noninterfering solvent, such as methanol, toluene, or methylene chloride, with or without an acid, such as p-toluenesulfonic acid or trifluoroacetic acid, with or without N-chlorosuccinimide and dimethyl sulfide, to afford the coupled product (221).

Compound (221) is cyclized under basic conditions with a copper (I) salt in an inert solvent according to the general procedure of Yang, et al., (Ref 8). The derivative (221) is treated with a base, such as sodium hydride, in an inert solvent, such as HMPA, at a temperature between 0 and 25°C. A copper (I) salt, such as copper (I) iodide, is added and the resultant mixture stirred at a temperature between 25 and 150°C for 1 to 48 hours to afford compound (222).

Compound (221) may also be cyclized according to the general procedure of Chen, et al., (Ref 10). The derivative (221) is treated with a base, such as sodium bicarbonate, and a palladium catalyst, such as $Pd(PPh_3)_4$, in an inert solvent, such as HMPA, at a temperature between 25 and 150°C to afford compound (222).

In a preferred method, intermediate (171) is treated with a transition metal catalyst, such as $Pd(OAc)_2(O\text{-}tol)_3P$ in the presence of a base such as triethylamine using a cosolvent of DMF/acetonitrile to prepare (222).

Compound (222) is N-alkylated with an appropriately substituted benzyl halide in the presence of a base, such as sodium hydride or potassium carbonate, in a noninterfering solvent, such as dimethylformamide or dimethylsulfoxide to afford ketone (223). In a two step, one pot process(222) is aromatized by treatment with acetic acid and palladium on carbon in a noninterfering solvent, such as carbitol or cymene, followed by treatment with hydrogen gas and palladium on carbon to cleave the nitrogen protecting group and produce the phenolic derivative (224).

The ester (224) is converted to the corresponding amide (225) under standard conditions with ammonia (preferably) or an ammonium salt, such as ammonium acetate, in an inert solvent, such as water or alcohol, preferably methanol, or with $MeClAlNH_2$ in an inert solvent, such as toluene, at a temperature between 0 to 110°C. Alkylation of the phenolic oxygen of compound 38 with an appropriate haloester, such as methyl bromoacetate, in the presence of a base, such as cesium carbonate, potassium or sodium carbonate, in an inert solvent, such as dimethylformamide or dimethylsulfoxide affords the ester-amide (226). Other haloesters, such as ethyl bromoacetate, propyl bromoacetate, butyl bromoacetate, and the like can also be used to prepare the corresponding esters.

Saponification of compound (226), with lithium hydroxide in an inert solvent, such as methanol-water, affords (227). The intermediate and final products may isolated and purified by conventional techniques such as chromatography or recrystallization. Regioisomeric products and intermediates can be separated by standard methods, such as, recrystallization or chromatography.

References:

10) L.-C. Chen et al., Synthesis 385 (1995)
11) S.-C. Yang et al., Heterocycles, 32, 2399 (1991)

The following example further illustrates the preparation of the compounds of this invention. The example is illustrative only and is not intended to limit the scope of the invention in any way.

Illustrative Example 1

2-(4-oxo-5-carboxamido-9-benzyl-9*H*-pyrido[3,4-*b*]indolyl)acetic acid hydrochloride

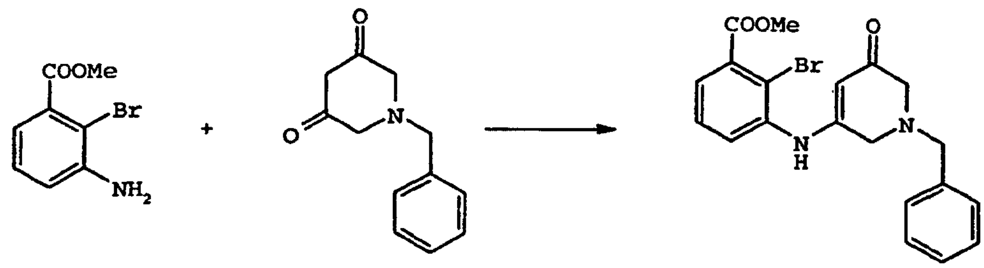

A. Preparation of N-[5-(1-benzyl-3-oxo-1,2,3,6-tetrahydropyridinyl)]-2-bromo-3-carbomethoxyaniline

To a mixture of 2-bromo-3-carbomethoxyaniline (12.0 g, 52.2 mmol) and pyridinium *p*-toluenesulfonate (13.8 g, 54.9 mmol) in 2:1 toluene/dioxane (300 mL) was added 1-benzyl-3,5-piperidinedione (13.0 g, 70.2 mmol, Chen, L. -C.; Yang, S.-C. *Heterocycles* **1990,** 31, 911-916). The apparatus was fitted with a Dean-Starke trap and the mixture refluxed for 10 h. The mixture was concentrated in vacuo and the residue dissolved in chloroform. This solution was washed three times with water, once with saturated sodium chloride solution, dried (sodium sulfate), filtered, and concentrated in vacuo to provide a dark oil. Chromatography (silica gel, chloroform to 4% methanol/96% chloroform) provided 2.0 g (9%) of the title product as a foam which could be crystallized form acetonitrile: mp 156-158 °C. $^1$H NMR ($CDCl_3$) d 7.55 (m, 2 H), 7.40 (m, 6 H), 5.55 (s, 1 H), 3.94 (s, 3 H), 3.85 (m, 2 H), 3.56 (m, 2 H), 3.30 (bs, 2 H); MS ES+ m/e 414.9 (p), 416.9 (p); IR (KBr, $cm^{-1}$) 3185, 2944, 1728, 1603, 1544, 1306.

| Elemental Analysis for $C_{20}H_{19}BrN_2O_3$: | | | |
|---|---|---|---|
| Calculated | C, 57.84; | H, 4.61; | N, 6.75. |
| Found | C, 58.13; | H, 4.49; | N, 6.91. |

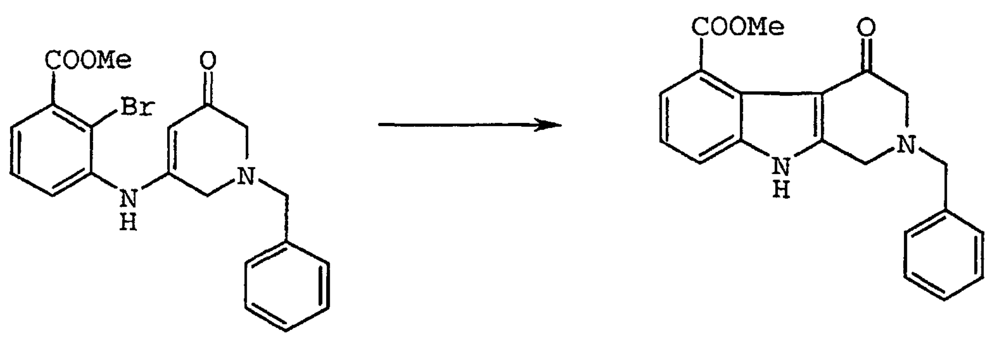

B. Preparation of 2-benzyl-4-oxo-5-carbomethoxy-1,2,3,4-tetrahydro-*9H*-pyrido[3,4-*b*]indole.

A mixture of N-[5-(1-benzyl-3-oxo-1,2,3,6-tetrahydropyridinyl)]-2-bromo-3-carbomethoxyaniline (2.07 g, 4.98 mmol), palladium(II) acetate (0.112 g, 0.499 mmol), tri-o-tolylphosphine (0.304 g, 0.999 mmol), triethylamine (1.3 mL, 9.3 mmol), and N,N-dimethylformamide (3 mL) in acetonitrile (12 mL) was placed in a tube and purged with argon. The tube was sealed and heated at 100 °C for 16 h. The mixture was cooled to room temperature, diluted with ethyl acetate, filtered, and the filtrate concentrated in vacuo to give a dark oil. Chromatography (silica gel, chloroform to 4% methanol/96% chloroform) provided 1.28 g (77%) of an oil which crystallized upon storing at 10 °C: recrystallized fro EtoAc/hexane mp 174-176 °C. $^1$H NMR ($CDCl_3$) d 9.25 (bs, 1 H), 7.38 (d, J = 9 Hz, 2 H), 7.30 (m, 5 H), 7.23 (t, J = 8 Hz, 1 H), 3.97 (s, 3 H), 3.75 (s, 2 H), 3.72 (s, 2 H), 3.61 (s, 2 H); MS ES+ m/e 335 (p + 1); IR (KBr, $cm^{-1}$) 3080, 1721,

1628, 1476, 1294, 1138.

| Elemental Analysis for $C_{20}H_{18}N_2O_3$: | | | |
|---|---|---|---|
| Calculated | C, 71.84; | H, 5.43; | N, 8.38. |
| Found | C, 72.06; | H, 5.31; | N, 8.31. |

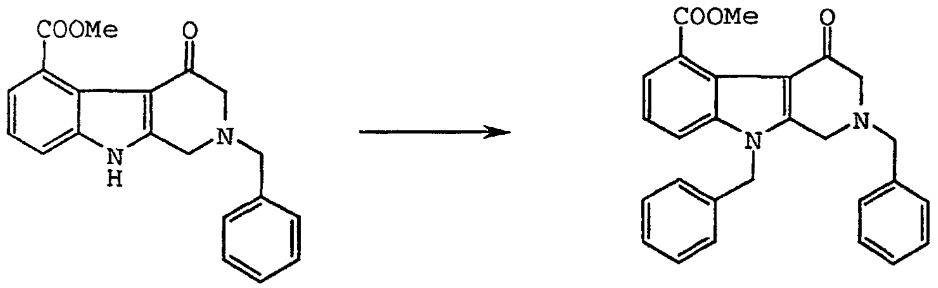

C. Preparation of 2,9-dibenzyl-4-oxo-5-carbomethoxy-1,2,3,4-tetrahydro-*9H*-pyrido[3,4-*b*]indole.

To a solution of 2-benzyl-4-oxo-5-carbomethoxy-1,2,3,4-tetrahydro-*9H*-pyrido[3,4-*b*]indole (0.928 g, 2.78 mmol) in dry tetrahydrofuran (5 mL) was added 60% sodium hydride in oil (111 mg). The resulting mixture was stirred at room temperature until gas evolution ceased. A solution of benzyl iodide (0.606 g, 2.78 mmol) in dry tetrahydrofuran (5 mL) was added to the reaction mixture and the resulting solution stirred at room temperature for 60 h. The mixture was diluted with methylene chloride and washed twice with saturated sodium chloride solution. The organic layer was dried (magnesium sulfate), filtered, and concentrated in vacuo. The residue was triturated with ethyl acetate resulting in a yellow precipitate (163 mg). The filtrate was concentrated in vacuo and chromatographed (silica gel, 5% methanol/ 95% methylene chloride) to provide an additional 580 mg of the title compound (743 mg total, 63%) as a crystalline solid: mp 198-199 °C. $^1H$ NMR ($CDCl_3$) d 7.43 (d, J = 7 Hz, 1 H), 7.36 (d, J = 8 Hz, 1 H), 7.25 (m, 9 H), 6.95 (m, 2 H), 5.24 (s, 2 H), 4.01 (s, 3 H), 3.78 (m, 4 H), 3.40 (bs, 2 H); MS EI+ m/e 425 (p + 1); IR (KBr, $cm^{-1}$) 1726, 1648, 1449, 1291, 1134, 1107.

| Elemental Analysis for $C_{27}H_{24}N_2O_3$: | | | |
|---|---|---|---|
| Calculated | C, 76.40; | H, 5.70; | N, 6.60. |
| Found | C, 76.11; | H, 5.45; | N, 6.54. |

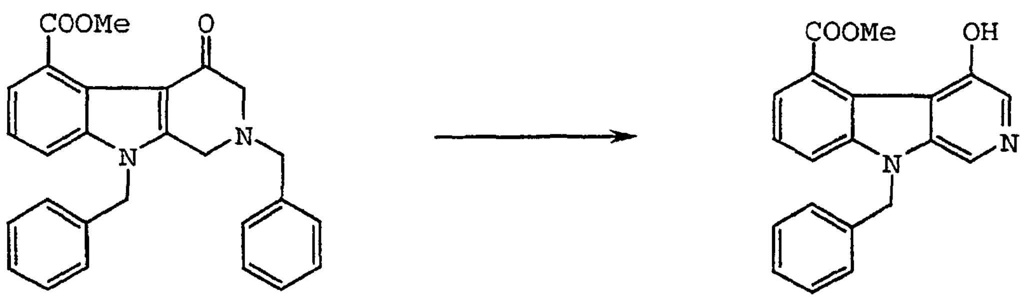

D. Preparation 4-hydroxy-5-carbomethoxy-9-benzyl-9H-pyrido[3,4-*b*]indole.

A mixture of of 2,9-dibenzyl-4-oxo-5-carbomethoxy-1,2,3,4-tetrahydro-*9H*-pyrido[3,4-*b*]indole (521 mg, 1.23 mmol) and 10% palladium-on-carbon (250 mg) in acetic acid (15 mL) was refluxed for 4 h. The reaction flask was cooled to room temperature and purged with nitrogen. The flask was placed under a positive pressure of hydrogen and heated at 75°C for 16 h. The mixture was cooled to room temperature, filtered, and concentrated in vacuo to provide an orange solid. Chromatography (silica gel, 4% methanol/96% methylene chloride) provided 271 mg (60%) of the title compound as a mono-hydrated yellow powder: mp >250 °C.

$^1H$ NMR ($CDCl_3$) d 8.46 (s, 1 H), 8.22 (s, 1 H), 8.09 (d, J = 8 Hz, 1 H), 7.70 (d, J = 8 Hz, 1 H), 7.56 (t, J = 8 Hz, 1 H), 7.23 (m, 3 H), 7.08 (m, 2 H), 5.60 (s, 2 H), 4.11 (s, 3 H); MS ES+ m/e 333 (p + 1).

| Elemental Analysis for $C_{20}H_{16}N_2O_3 \cdot H_2O$: | | | |
|---|---|---|---|
| Calculated | C, 68.60; | H, 4.98; | N, 7.91. |
| Found | C, 68.56; | H, 5.18; | N, 8.00. |

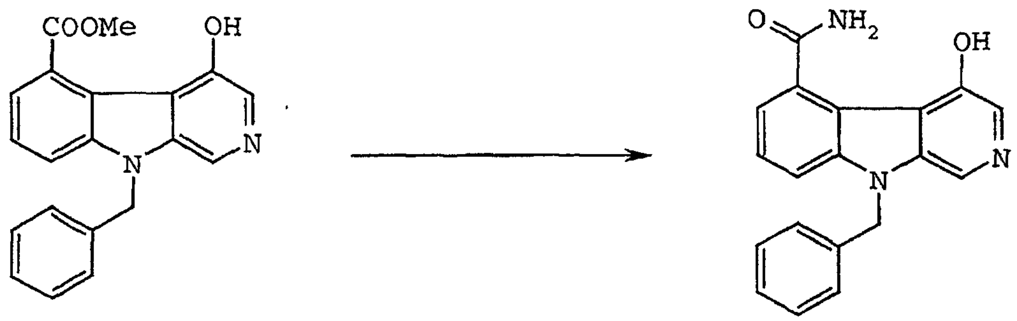

E. Preparation of 4-hydroxy-5-carboxamido-9-benzyl-9H-pyrido[3,4-*b*]indole.

4-Hydroxy-5-carbomethoxy-9-benzyl-*9H*-pyrido[3,4-*b*]indole (200 mg, 0.618 mmol) was dissolved in a solution of 2M methanolic ammonia (10 mL) and placed in an open tube. The solution was saturated with gaseous ammonia for 10 min. The tube was sealed and heated at 60-65 °C for 8 h. The reaction mixture was cooled to room temperature and the resulting precipitate was collected in vacuo to provide 0.12 g (61%) of the title compound as a yellow solid: mp >250 °C. $^1H$ NMR (DMSO-$d_6$) d 10.99 (s, 1 H, -OH), 8.99 (bs, 1 H, -NH), 8.59 (s, 1 H), 8.55 (bs, 1 H, -NH), 7.96 (d, J = 7 Hz, 1 H), 7.94 (s, 1 H), 7.64 (t, J = 8 Hz, 1 H), 7.57 (d, J = 7 Hz, 1 H), 7.22 (m, 3H), 7.12 (d, J - 7 Hz, 2H), 5.80 (s, 2 H); MS ES+ m/e 318 (p + 1).

| Elemental Analysis for $C_{19}H_{15}N_3O_2$: | | | |
|---|---|---|---|
| Calculated | C, 71.91; | H, 4.76; | N, 13.24. |
| Found | C, 72.20; | H, 4.57; | N, 13.48. |

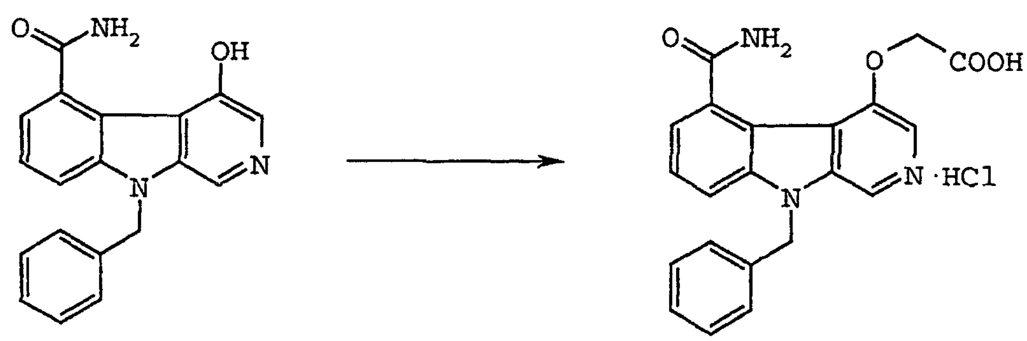

F. Preparation of 2-(4-oxo-5-carboxamido-9-benzyl-9H-pyrido[3,4-*b*]indolyl)acetic acid hydrochloride.

A mixture of 4-hydroxy-5-carboxamido-9-benzyl-9H-pyrido[3,4-*b*]indole (57 mg, 0.18 mmol), methyl bromoacetate (51 mL, 0.54 mmol), and cesium carbonate (114 mg, 0.349 mmol) in N,N-dimethylformamide (2 mL) was stirred at room temperature for 45 min. The mixture was treated with a minimum of water and methanol and concentrated in vacuo. The residue was dissolved in 1M aqueous lithium hydroxide (0.5 mL) and stirred at room temperature for 1 h. The mixture was concentrated in vacuo. The residue was dissolved in dilute aqueous hydrochloric acid and purified

via reverse-phase HPLC, followed by lyophilization, to provide 28.5 mg (38%) of the title product. $^1H$ NMR (DMSO-$d_6$) d 12.85 (bs, 1 H), 9.41 (s, 1 H), 9.11 (s, 1 H), 8.66 (s, 1 H), 8.30 (s, 1 H), 8.10 (d, J = 8 Hz, 1 H), 7.85 (t, J = 8 Hz, 1 H), 7.76 (d, J = 7 Hz, 1 H), 7.27 (m, 3 H), 7.19 (m, 2 H), 5.88 (s, 2 H), 5.37 (s, 2 H); MS ES+ m/e 375 (p + 1).

| Elemental Analysis for $C_{21}H_{17}N_3O_4^-$ HCl$^-$ 0.5$H_2O$: | | | |
|---|---|---|---|
| Calculated | C, 60.58; | H, 4.47; | N, 10.09. |
| Found | C, 60.39; | H, 4.35; | N, 9.69. |

Therapeutic Use of Tricyclic Compounds

The compounds described herein are believed to achieve their beneficial therapeutic action principally by direct inhibition of human $sPLA_2$, and not by acting as antagonists for arachidonic acid, nor other active agents below arachidonic acid in the arachidonic acid cascade, such as 5-lipoxygenases, cyclooxygenases, etc.

The use of the invention for inhibiting $sPLA_2$ mediated release of fatty acids comprises contacting $sPLA_2$ with an therapeutically effective amount of a compound of the invention or its salt.

The compounds of the invention are useful in a method of treating a mammal (e.g., a human) to alleviate the pathological effects of septic shock, adult respiratory distress syndrome, pancreatitus, trauma, bronchial asthma, allergic rhinitis, and rheumatoid arthritis; wherein the method comprises administering to the mammal a compound of formula (I) in a therapeutically effective amount. A "therapeutically effective" amount is an amount sufficient to inhibit $sPLA_2$ mediated release of fatty acid and to thereby inhibit or prevent the arachidonic acid cascade and its deleterious products. The therapeutic amount of compound of the invention needed to inhibit $sPLA_2$ may be readily determined by taking a sample of body fluid and assaying it for $sPLA_2$ content by conventional methods.

Throughout this document, the person or animal to be treated will be described as a "mammal", and it will be understood that the most preferred subject is a human. However it must be noted that the study of adverse conditions of the central nervous system in non-human animals is only now beginning, an that some instances of such treatments are coming into use. Accordingly, use of the present compounds in non-human animals is contemplated. It will be understood that the dosage ranges for other animals will necessarily be quite different from the doses administered to humans, and accordingly that the dosage ranges described be recalculated. For example, a small dog may be only 1/10$^{th}$ of a typical human's size, and it will therefore be necessary for a much smaller dose to be used. The determination of an effective amount for a certain non-human animal is carried out in the same manner described below in the case of humans, and veterinarians are well accustomed to such determinations.

Pharmaceutical Formulations of the Invention

As previously noted the compounds of this invention are useful for inhibiting $sPLA_2$ mediated release of fatty acids such as arachidonic acid. By the term, "inhibiting" is meant the prevention or therapeutically significant reduction in release of $sPLA_2$ initiated fatty acids by the compounds of the invention. By "pharmaceutically acceptable" it is meant the carrier, diluent or excipient must be compatible with the other ingredients of the formulation and not deleterious to the recipient thereof.

In general, the compounds of the invention are most desirably administered at a dose that will generally afford effective results without causing any serious side effects and can be administered either as a single unit dose, or if desired, the dosage may be divided into convenient subunits administered at suitable times throughout the day.

The specific dose of a compound administered according to this invention to obtain therapeutic or prophylactic effects will, of course, be determined by the particular circumstances surrounding the case, including, for example, the route of administration, the age, weight and response of the individual patient, the condition being treated and the severity of the patient's symptoms. Typical daily doses will contain a non-toxic dosage level of from about 0.01 mg/kg to about 50 mg/kg of body weight of an active compound of this invention.

Preferably the pharmaceutical formulation is in unit dosage form. The unit dosage form can be a capsule or tablet itself, or the appropriate number of any of these. The quantity of active ingredient in a unit dose of composition may be varied or adjusted from about 0.1 to about 1000 milligrams or more according to the particular treatment involved. It may be appreciated that it may be necessary to make routine variations to the dosage depending on the age and condition of the patient. The dosage will also depend on the route of administration.

A "chronic" condition means a deteriorating condition of slow progress and long continuance. As such, it is treated when it is diagnosed and continued throughout the course of the disease. An "acute" condition is an exacerbation of short course followed by a period of remission. In an acute event, compound is administered at the onset of symptoms and discontinued when the symptoms disappear.

Pancreatitis, trauma-induced shock, bronchial asthma, allergic rhinitis and rheumatoid arthritis may occur as

an acute event or a chronic event. Thus, the treatment of these conditions contemplates both acute and chronic forms. Septic shock and adult respiratory distress, on the other hand, are acute conditions treated when diagnosed.

The compound can be administered by a variety of routes including oral, aerosol, rectal, transdermal, subcutaneous, intravenous, intramuscular, and intranasal.

Pharmaceutical formulations of the invention are prepared by combining (e.g., mixing) a therapeutically effective amount of the compounds of the invention together with a pharmaceutically acceptable carrier or diluent therefor. The present pharmaceutical formulations are prepared by known procedures using well known and readily available ingredients.

In making the compositions of the present invention, the active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, or can be in the form of tablets, pills, powders, lozenges, elixirs, suspensions, emulsions, solutions, syrups, aerosols (as a solid or in a liquid medium), or ointment, containing, for example, up to 10% by weight of the active compound. The compounds of the present invention are preferably formulated prior to administration.

For the pharmaceutical formulations any suitable carrier known in the art can be used. In such a formulation, the carrier may be a solid, liquid, or mixture of a solid and a liquid. Solid form formulations include powders, tablets and capsules. A solid carrier can be one or more substances which may also act as flavoring agents, lubricants, solubilisers, suspending agents, binders, tablet disintegrating agents and encapsulating material.

Tablets for oral administration may contain suitable excipients such as calcium carbonate, sodium carbonate, lactose, calcium phosphate, together with disintegrating agents, such as maize, starch, or alginic acid, and/or binding agents, for example, gelatin or acacia, and lubricating agents such as magnesium stearate, stearic acid, or talc.

In powders the carrier is a finely divided solid which is in admixture with the finely divided active ingredient. In tablets the active ingredient is mixed with a carrier having the necessary binding properties in suitable proportions and compacted in the shape and size desired. The powders and tablets preferably contain from about 1 to about 99 weight percent of the active ingredient which is the novel compound of this invention. Suitable solid carriers are magnesium carbonate, magnesium stearate, talc, sugar lactose, pectin, dextrin, starch, gelatin, tragacanth, methyl cellulose, sodium carboxymethyl cellulose, low melting waxes, and cocoa butter.

Sterile liquid form formulations include suspensions, emulsions, syrups and elixirs.

The active ingredient can be dissolved or suspended in a pharmaceutically acceptable carrier, such as sterile water, sterile organic solvent or a mixture of both. The active ingredient can often be dissolved in a suitable organic solvent, for instance aqueous propylene glycol. Other compositions can be made by dispersing the finely divided active ingredient in aqueous starch or sodium carboxymethyl cellulose solution or in a suitable oil.

The following pharmaceutical formulations 1 through 8 are illustrative only and are not intended to limit the scope of the invention in any way. "Active ingredient", refers to a compound according to Formula (III) or a pharmaceutically acceptable salt, solvate, or prodrug thereof.

Formulation 1

Hard gelatin capsules are prepared using the following ingredients:

| | Quantity (mg/capsule) |
|---|---|
| 2-[4-oxo-5-carboxamido-9-(4-methylbenzyl)-9*H* -pyrido[3,4-*b* ]indolyl]acetic acid | 250 |
| Starch, dried | 200 |
| Magnesium stearate | 10 |
| Total | 460 mg |

Formulation 2

A tablet is prepared using the ingredients below:

| | Quantity (mg/tablet) |
|---|---|
| 2-[4-oxo-5-carboxamido-9-[4-(trifluoromethyl)benzyl]-9*H*-pyrido[3,4-*b*]indolyl]acetic acid | 250 |
| Cellulose, microcrystalline | 400 |
| Silicon dioxide, fumed | 10 |
| Stearic acid | 5 |
| Total | 665 mg |

The components are blended and compressed to form tablets each weighing 665 mg

Formulation 3

An aerosol solution is prepared containing the following components:

| | Weight |
|---|---|
| 2-[4-oxo-5-carboxamido-9-(3-benzoylbenzyl)-9*H* -pyrido-*b*]indolyl]acetic acid | 0.25 |
| Ethanol | 25.75 |
| Propellant 22 (Chlorodifluoromethane) | 74.00 |
| Total | 100.00 |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

Formulation 4

Tablets, each containing 60 mg of active ingredient, are made as follows:

| | |
|---|---|
| 2-[4-oxo-5-carboxamido-9-(2,4,6-trifluorobenzyl)-9*H*-pyrido[3,4-*b*]indolyl]acetic acid | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The aqueous solution containing polyvinylpyrrolidone is mixed with the resultant powder, and the mixture then is passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Formulation 5

Capsules, each containing 80 mg of active ingredient, are made as follows:

| | |
|---|---|
| 2-[4-oxo-5-carboxamido-9-(2-fluorobenzyl)-9*H*-pyrido[3,4-*b*]indolyl]acetic acid tetrahydrocarbazole-4-carboxamide | 80 mg |
| Starch | 59 mg |
| Microcrystalline cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

Formulation 6

Suppositories, each containing 225 mg of active ingredient, are made as follows:

| | |
|---|---|
| 2-[4-oxo-5-carboxamido-9-pentafluorobenzyl-9*H*-pyrido[3,4-*b*]indolyl]acetic acid | 225 mg |
| Saturated fatty acid glycerides | 2,000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

<u>Formulation 7</u>

Suspensions, each containing 50 mg of active ingredient per 5 ml dose, are made as follows:

| | |
|---|---|
| 2-[4-oxo-5-carboxamido-9-(3,4,5-trimethoxybenzyl)-9*H*-pyrido [3,4-*b*]indolyl]acetic acid | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

<u>Formulation 8</u>

An intravenous formulation may be prepared as follows:

| | |
|---|---|
| 2-[4-oxo-5-carboxamido-9-(3,5-difluorobenzyl)-9*H*-pyrido[3,4-*b*]indolyl]acetic acid | 100 mg |
| Isotonic saline | 1,000 ml |

The solution of the above ingredients generally is administered intravenously to a subject at a rate of 1 ml per minute.

<u>Assay Experiments</u>

<u>Assay Example 1</u>

The following chromogenic assay procedure was used to identify and evaluate inhibitors of recombinant human secreted phospholipase $A_2$. The assay described herein has been adapted for high volume screening using 96 well microtiter plates. A general description of this assay method is found in the article, "Analysis of Human Synovial Fluid Phospholipase $A_2$ on Short Chain Phosphatidylcholine-Mixed Micelles: Development of a Spectrophotometric Assay Suitable for a Microtiterplate Reader", by Laure J. Reynolds, Lori L. Hughes, and Edward A Dennis, <u>Analytical Biochemistry</u>, 204, pp. 190-197, 1992 (the disclosure of which is incorporated herein by reference):

Reagents:

REACTION BUFFER -

$CaCl_2.2H_2O$ (1.47 g/L)
KCl (7.455 g/L)
Bovine Serum Albumin (fatty acid free) (1 g/L)
(Sigma A-7030, product of Sigma Chemical Co. St. Louis MO, USA)
TRIS HCl (3.94 g/L)

pH 7.5 (adjust with NaOH)

ENZYME BUFFER -

0.05 $NaOAc.3H_2O$, pH 4.5
0.2 NaCl
Adjust pH to 4.5 with acetic acid

DTNB - 5,5'-dithiobis-2-nitrobenzoic acid
RACEMIC DIHEPTANOYL THIO - PC

racemic 1,2-bis(heptanoylthio) -1,2-dideoxy-*sn*-glycero-3-phosphorylcholine
TRITON X-100™ prepare at 6.249 mg/ml in reaction buffer to equal 10uM
TRITON X-100™ is a polyoxy ethylene non-ionic detergent supplied by Pierce Chemical Company,
3747 N. Meridian Road, Rockford, Illinois 61101.

REACTION MIXTURE -

A measured volume of racemic dipheptanoyl thio PC supplied in chloroform at a concentration of 100 mg/ml is taken to dryness and redissolved in 10 millimolar TRITON X-100™ nonionic detergent aqueous solution. Reaction Buffer is added to the solution, then DTNB to give the Reaction Mixture.

The reaction mixture thus obtained contains 1mM diheptanoly thio-PC substrate, 0.29 mm Triton X-100™ detergent, and 0.12 mm DTMB in a buffered aqueous solution at pH 7.5.

Assay Procedure:

1. Add 0.2 ml reaction mixture to all wells;
2. Add 10 ul test compound (or solvent blank) to appropriate wells, mix 20 seconds;
3. Add 50 nanograms of $sPLA_2$ (10 microliters) to appropriate wells;
4. Incubate plate at 40°C for 30 minutes;
5. Read absorbance of wells at 405 nanometers with an automatic plate reader.

All compounds were tested in triplicate. Typically, compounds were tested at a final concentration of 5 ug/ml. Compounds were considered active when they exhibited 40% inhibition or greater compared to uninhibited control reactions when measured at 405 nanometers. Lack of color development at 405 nanometers evidenced inhibition. Compounds initially found to be active were reassayed to confirm their activity and, if sufficiently active, $IC_{50}$ values were determined. Typically, the $IC_{50}$ values were determined by diluting test compound serially two-fold such that the final concentration in the reaction ranged from 45 ug/mL to 0.35 ug/ml. More potent inhibitors required significantly greater dilution. In all cases, % inhibition measured at 405 nanometers generated by enzyme reactions containing inhibitors relative to the uninhibited control reactions was determined. Each sample was titrated in triplicate and result values were averaged for plotting and calculation of $IC_{50}$ values. $IC_{50}$ were determined by plotting log concentration versus inhibition values in the range from 10-90% inhibition.

Compounds of the instant invention were tested in Assay Example 1 and were found to be effective at concentrations of less than 100µM.

Assay Example 2

Method:

Male Hartley strain guinea pigs (500-700g) were killed by cervical dislocation and their heart and lungs removed intact and placed in aerated (95% $O_2$:5% $CO_2$) Krebs buffer. Dorsal pleural strips (4x1x25mm) were dissected from intact parenchymal segments (8x4x25mm) cut parallel to the outer edge of the lower lung lobes. Two adjacent pleural strips, obtained from a single lobe and representing a single tissue sample, were tied at either end and independently attached to a metal support rod. One rod was attached to a Grass force-displacement transducer Model FTO3C, product of Grass Medical Instruments Co., Qincy, MA, USA). Changes in isometric tension were displayed on a monitor and thermal recorder (product of Modular Instruments, Malvern, PA). All tissues were placed in 10 ml jacketed

tissue baths maintained at 37°C. The tissue baths were continuously aerated and contained a modified Krebs solution of the following composition (millimolar) NaCl, 118.2; KCl, 4.6; $CaCl_2 \cdot 2H_2O$, 2.5; $MgSO_4 \cdot 7H_2O$, 1.2; $NaHCO_3$, 24.8; $KH_2PO_4$, 1.0; and dextrose, 10.0. Pleural strips from the opposite lobes of the lung were used for paired experiments. Preliminary data generated from tension/response curves demonstrated that resting tension of 800mg was optimal. The tissues were allowed to equilibrate for 45 min. as the bath fluid was changed periodically.

Cumulative concentration-response curves:

Initially tissues were challenged 3 times with KCl (40 mM) to test tissue viability and to obtain a consistent response. After recording the maximal response to KCl, the tissues were washed and allowed to return to baseline before the next challenge. Cumulative concentration-response curves were obtained from pleural strips by increasing the agonist concentration ($sPLA_2$) in the tissue bath by half-$log_{10}$ increments while the previous concentration remained in contact with the tissues (Ref.1, supra.). Agonist concentration was increased after reaching the plateau of the contraction elicited by the preceding concentration. One concentration-response curve was obtained from each tissue. To minimize variability between tissues obtained from different animals, contractile responses were expressed as a percentage of the maximal response obtained with the final KCl challenge. When studying the effects of various drugs on the contractile effects of $sPLA_2$, the compounds and their respective vehicles were added to the tissues 30 minutes prior to starting the $sPLA_2$ concentration-response curves.

Statistical analysis:

Data from different experiments were pooled and presented as a percentage of the maximal KCl responses (mean ± S.E.). To estimate the drug induced rightward shifts in the concentration response curves, the curves were analyzed simultaneously using statistical nonlinear modeling methods similar to those described by Waud (1976), Equation 26, p. 163, (Ref.2). The model includes four parameters: the maximum tissue response which was assumed the same for each curve, the $ED_{50}$ for the control curve, the steepness of the curves, and the $pA_2$, the concentration of antagonist that requires a two-fold increase in agonist to achieve an equivalent response. The Schild slope was determined to be 1, using statistical nonlinear modeling methods similar to those described by Waud (1976), Equation 27, p. 164 (Ref. 2). The Schild slope equal to 1 indicates the model is consistent with the assumptions of a competitive antagonist; therefore, the pA2 may be interpreted as the apparent $K_B$, the dissociation constant of the inhibitor.

To estimate the drug-induced suppression of the maximal responses, $sPLA_2$ responses (10 ug/ml) were determined in the absence and presence of drug, and percent suppression was calculated for each pair of tissues. Representative examples of inhibitory activities are presented in Table 2, below.

Ref. 1 - Van, J.M.: Cumulative dose-response curves. II. Technique for the making of dose-response curves in isolated organs and the evaluation of drug parameters. Arch. Int. Pharmacodyn. Ther., 143: 299-330, 1963.
Ref. 2 - Waud, D.: Analysis of dose-response relationships. in Advances in General and Cellular Pharmacology eds Narahashi, Bianchi: 1:145-178, 1976.

Compounds of the instant invention were tested in Assay Example 2 and were found to be effective at concentrations below 20μM.

Assay Example 3

$sPLA_2$ Transgenic Mice Assay

Materials & Methods

The mice utilized in these studies were mature, 6-8 month old, $ZnSO_4$-stimulated, hemizygous line 2608[a] transgenic mice (Fox et. al. 1996). Transgenic mice from this line express human $sPLA_2$ in the liver and other tissues and typically achieve levels of human $sPLA_2$ in their circulation of approximately 173 ± 10 ng/ml when maximally stimulated with $ZnSO_4$ (Fox, et al. 1996). The mice were housed under constant humidity and temperature and received food and water ad libitum. Animal room lighting was maintained on a 12-hour light/dark cycle and all experiments were performed at the same time of the day during the early morning light period.

For intravenous testing, compounds or vehicle were administered as an IV bolus via the tail vein in a volume of 0.15 ml. Vehicle consisted of 1-5% dimethylsulfoxide, 1-5% ethanol and 10-30% polyethylene glycol 300 in $H_2O$; the concentrations of these ingredients were adjusted according to the solubility of the compound. Mice were bled retro-orbitally prior to drug or vehicle administration and 30 minutes, 2 and 4 hours thereafter. Three to six mice were

used for each dose. $PLA_2$ catalytic activity in the serum was assayed with a modified phosphatidylcholine/deoxycholine mixed micelle assay (Fox, *et al.* 1996, Schadlich, *et al.,* 1987) utilizing 3 mM sodium deoxycholate and 1 mM 1-palmitoyl-2-oleoyl-sn-glycero-3-phosphocholine.

For oral testing, compounds were dissolved in 1-5% ethanol/10-30% polyethylene glycol 300 in $H_2O$ or were suspended in 5% dextrose in $H_2O$ and administered by oral gavage. Serum was prepared from retro-orbital blood and assayed for $PLA_2$ catalytic activity as above.

References

Fox, N., M. Song, J. Schrementi, J. D. Sharp, D. L.
White, D. W. Snyder, L. W. Hartley, D. G. Carlson, N. J.
Bach, R. D. Dillard, S. E. Draheim, J. L. Bobbitt, L.
Fisher and E. D. Mihelich. 1996. Eur. J. Pharmacol. 308: 195.
Schadlich, H.R., M. Buchler, and H. G. Beger, 1987, J. Clin. Chem. Clin. Biochem. 25, 505.

Compounds of the instant invention were tested in Assay Example 3 and were found to be effective.

While the present invention has been illustrated above by certain specific embodiments, it is not intended that these specific examples should limit the scope of the invention as described in the appended claims.

## Claims

**1.** A compound which is selected from the group consisting of
2-[4-oxo-5-carboxamido-9-(2-methylbenzyl)-9H-pyrido[3,4-*b*]indolyl]acetic acid;
2-[4-oxo-5-carboxamido-9-(3-methylbenzyl)-*9H*-pyrido[3,4-*b*]indolyl]acetic acid;
2-[4-oxo-5-carboxamido-9-(4-methylbenzyl)-9H-pyrido[3,4-*b*]indolyl]acetic acid;
2-[4-oxo-5-carboxamido-9-(4-*tert*-butylbenzyl)-*9H*-pyrido[3,4-*b*]indolyl]acetic acid;
2-[4-oxo-5-carboxamido-9-pentafluorobenzyl-*9H*-pyrido[3,4-*b*]indolyl]acetic acid;
2-[4-oxo-5-carboxamido-9-(2-fluorobenzyl)-*9H*-pyrido[3,4-*b*]indolyl]acetic acid;
2-[4-oxo-5-carbaxamido-9-(3-fluorobenzyl)-*9H*-pyrido[3,4-*b*]indolyl]acetic acid;
2-[4-oxo-5-carboxamido-9-(4-fluorobenzyl)-9H-pyrido[3,4-*b*]indolyl]acetic acid;
2-[4-oxo-5-carboxamido-9-(2,6-difluorobenzyl)-*9H*-pyrido[3,4-*b*]indolyl]acetic acid;
2-[4-oxo-5-carboxamido-9-(3,4-difluorobenzyl)-*9H*-pyrido[3,4-*b*]indolyl]acetic acid;
2-[4-oxo-5-carboxamido-9-(2,5-difluorobenzyl)-*9H*-pyrido[3,4-*b*]indolyl]acetic acid;
2-[4-oxo-5-carboxamido-9-(3,5-difluorobenzyl)-*9H*-pyrido[3,4-*b*]indolyl]acetic acid;
2-[4-oxo-5-carboxamido-9-(2,4-difluorobenzyl)-*9H*-pyrido[3,4-*b*]indolyl]acetic acid;
2-[4-oxo-5-carboxamido-9-(2,3-difluorobenzyl)-*9H*-pyrido[3,4-*b*]indolyl]acetic acid;
2-[4-oxo-5-carboxamido-9-[2-(trifluoromethyl)benzyl]-*9H*-pyrido[3,4-*b*]indolyl]acetic acid;
2-[4-oxo-5-carboxamido-9-[3-(trifluoromethyl)benzyl]-*9H*-pyrido[3,4-*b*]indolyl]acetic acid;
2-[4-oxo-5-carboxamido-9-[4-(trifluoromethyl)benzyl]-*9H*-pyrido[3,4-*b*]indolyl]acetic acid;
2-[4-oxo-5-carboxamido-9-[3,5-bis(trifluoromethyl)benzyl]-*9H*-pyrido[3,4-*b*]indolyl]acetic acid;
2-[4-oxo-5-carboxamido-9-[2,4-bis(trifluoromethyl)benzyl]-*9H*-pyrido[3,4-*b*]indolyl]acetic acid;
2-[4-oxo-5-carboxamido-9-(a-methylnaphthyl)-*9H*-pyrido[3,4-*b*]indolyl]acetic acid;
2-[4-oxo-5-carboxamido-9-(b-methylnaphthyl)-*9H*-pyrido[3,4-*b*]indolyl]acetic acid;
2-[4-oxo-5-carboxamido-9-(3,5-dimethylbenzyl)-*9H*-pyrido[3, 4-*b*]indolyl]acetic acid;
2-[4-oxo-5-carboxamido-9-(2,4-dimethylbenzyl)-*9H*-pyrido[3,4-*b*]indolyl]acetic acid;
2-[4-oxo-5-carboxamido-9-(2-phenylbenzyl)-*9H*-pyrido[3,4-*b*]indolyl]acetic acid;
2-[4-oxo-5-carboxamido-9-(3-phenylbenzyl)-*9H*-pyrido(3,4-*b*]indolyl]acetic acid;
2-[4-oxo-5-carboxamido-9-(4-phenylbenzyl)-*9H*-pyrido[3,4-*b*]indolyl]acetic acid;
2-[4-oxo-5-carboxamido-9-(1-fluorenylmethyl)-*9H*-pyrido[3,4-*b*]indolyl]acetic acid;
2-[4-oxo-5-carboxamido-9-(2-fluoro-3-methylbenzyl)-*9H*-pyrido[3,4-*b*]indolyl]acetic acid;
2-[4-oxo-5-carboxamido-9-(3-benzoylbenzyl)-*9H*-pyrido[3,4-*b*]indolyl]acetic acid;
2-[4-oxo-5-carboxamido-9-(2-phenoxybenzyl)-9H-pyrido[3,4-*b*]indolyl]acetic acid;
2-[4-oxo-5-carboxamido-9-(3-phenoxybenzyl)-*9H*-pyrido[3,4-*b*]indolyl]acetic acid;
2-[4-oxo-5-carboxamido-9-(4-phenoxybenzyl)-*9H*-pyrido[3,4-*b*]indolyl]acetic acid;
2-[4-oxo-5-carboxamido-9-[3-[2-(fluorophenoxy)benzyl]]-*9H*-pyrido[3,4-*b*]indolyl]acetic acid;

2-[4-oxo-5-carboxamido-9-[3-[4-(fluorophenoxy)benzyl]]-*9H*-pyrido[3,4-*b*]indolyl]acetic acid;
2-[4-oxo-5-carboxamido-9-[2-fluoro-3-(trifluoromethyl)benzyl]-*9H*-pyrido[3,4-*b*]indolyl]acetic cid;
2-[4-oxo-5-carboxamido-9-[2-fluoro-4-(trifluoromethyl)benzyl]-*9H*-pyrido[3,4-*b*]indolyl]acetic acid;
2-[4-oxo-5-carboxamido-9-[2-fluoro-5-(trifluoromethyl)benzyl]-*9H*-pyrido[3,4-b]indolyl]acetic acid;
2-[4-oxo-5-carboxamido-9-[3-fluoro-5-(trifluoromethyl)benzyl]-*9H*-pyrido[3,4-*b*]indolyl]acetic acid;
2-[4-oxo-5-carboxamido-9-[4-fluoro-2-(trifluoromethyl)benzyl]-*9H*-pyrido[3,4-*b*]indolyl]acetic acid;
2-[4-oxo-5-carboxamido-9-[4-fluoro-3-(trifluoromethyl)benzyl]-*9H*-pyrido[3,4-*b*]indolyl]acetic acid;
2-[4-oxo-5-carboxamido-9-[2-fluoro-6-(trifluoromethyl)benzyl]-*9H*-pyrido[3,4-*b*]indolyl]acetic acid;
2-[4-oxo-5-carboxamido-9-(2,3,6-trifluorobenzyl)-*9H*-pyrido[3,4-*b*]indolyl]acetic acid;
2-[4-oxo-5-carboxamido-9-(2,3,5-trifluorobenzyl)-*9H*-pyrido[3,4-*b*]indolyl]acetic acid;
2-[4-oxo-5-carboxamido-9-(2,4,5-trifluorobenzyl)-*9H*-pyrido[3,4-*b*]indolyl]acetic acid;
2-[4-oxo-5-carboxamido-9-(2,4,6-trifluorobenzyl)-*9H*-pyrido[3,4-*b*]indolyl]acetic acid;
2-[4-oxo-5-carboxamido-9-(2,3,4-trifluorobenzyl)-*9H*-pyrido[3,4-*b*]indolyl]acetic acid;
2-[4-oxo-5-carboxamido-9-(3,4,5-trifluorobenzyl)-*9H*-pyrido[3,4-*b*]indolyl]acetic acid;
2-[4-oxo-5-carboxamido-9-[3-(trifluoromethoxyl)benzyl]-*9H*-pyrido[3,4-*b*]indolyl]acetic acid;
2-[4-oxo-5-carboxamido-9-[4-(trifluoromethoxyl)benzyl]-*9H*-pyrido[3,4-*b*]indolyl]acetic acid;
2-[4-oxo-5-carboxamido-9-[4-methoxy(tetrafluoro)benzyl]-*9H*-pyrido[3,4-*b*]indolyl]acetic acid;
2-[4-oxo-5-carboxamido-9-(2-methoxybenzyl)-*9H*-pyrido[3,4-*b*]indolyl]acetic acid;
2-[4-oxo-5-carboxamido-9-(3-methoxybenzyl)-9H-pyrido[3,4-*b*]indolyl]acetic acid;
2-[4-oxo-5-carboxamido-9-(4-methoxybenzyl)-9H-pyrido[3,4-b]indolyl]acetic acid;
2-[4-oxo-5-carboxamido-9-(4-ethylbenzyl)-*9H*-pyrido[3,4-*b*]indolyl]acetic acid;
2-[4-oxo-5-carboxamido-9-(4-isopropylbenzyl)-*9H*-pyrido[3,4-*b*]indolyl]acetic acid;
2-[4-oxo-5-carboxamido-9-(3,4,5-trimethoxybenzyl)-*9H*-pyrido[3,4-*b*]indolyl]acetic acid;
2-[4-oxo-5-carboxamido-9-(3,4-methylenedioxybenzyl)-9H-pyrido[3,4-b]indolyl]acetic acid;
2-[4-oxo-5-carboxamido-9-(4-methoxy-3-methylbenzyl)-9*H*-pyrido[3,4-b]indolyl]acetic acid;
2-[4-oxo-5-carboxamido-9-(3,5-dimethoxybenzyl)-9*H*-pyrido[3,4-*b*]indolyl]acetic acid;
2-[4-oxo-5-carboxamido-9-(2,5-dimethoxybenzyl)-9*H*-pyrido[3,4-*b*]indolyl]acetic acid;
2-[4-oxo-5-carboxamido-9-(4-ethoxybenzyl)-9*H*-pyrido[3,4-*b*]indolyl]acetic acid;
2-[4-oxo-5-carboxamido-9-(cyclohexylmethyl)-*9H*-pyrido[3,4-*b*]indolyl]acetic acid;
2-[4-oxo-5-carboxamido-9-(cyclopentylmethyl)-*9H*-pyrido[3,4-*b*]indolyl]acetic acid;
2-[4-oxo-5-carboxamido-9-ethyl-9H-pyrido[3,4-*b*]indolyl]acetic acid;
2-[4-oxo-5-carboxamido-9-(1-propyl)-9H-pyrido[3,4-*b*]indolyl]acetic acid;
2-[4-oxo-5-carboxamido-9-(2-propyl)-*9H*-pyrido[3,4-*b*]indolyl]acetic acid;
2-[4-oxo-5-carboxamido-9- (1-butyl) -9*H*-pyrido[3,4-*b*]indolyl]acetic acid;
2-[4-oxo-5-carboxamido-9-(2-butyl)-*9H*-pyrido[3,4-b]indolyl]acetic acid;
2-[4-oxo-5-carboxamido-9-isobutyl-*9H*-pyrido[3,4-b]indolyl]acetic acid;
2-[4-oxo-5-carboxamido-9-[2-(1-phenylethyl)]-*9H*-pyrrido[3,4-*b*]indolyl]acetic acid;
2-[4-oxo-5-carboxamido-9-[3-(1-phenylpropyl)]-*9H*-pyrido[3,4-*b*]indolyl]acetic acid;
2-[4-oxo-5-carboxamido-9-[4-(1-phenylbutyl)]-9*H*-pyrido[3,4-*b*]indolyl]acetic acid;
2-[4-oxo-5-carboxamido-9-(1-pentyl)-9*H*-pyrido[3,4-*b*]indolyl]acetic acid; and
2-[4-oxo-5-carboxamido-9-(1-hexyl)-*9H*-pyrido[3,4-b]indolyl]acetic acid;
or a pharmaceutically acceptable racemate, solvate, tautomer, optical isomer, prodrug derivative selected from the methyl, ethyl, propyl, isopropyl, butyl, sec-butyl, tert-butyl, morpholinoethyloxy, diethylglycolamide or diethylaminocarbonylmethoxy ester thereof or salt thereof.

**2.** A pharmaceutical formulation comprising a compound as claimed in Claim 1 together with a pharmaceutically acceptable carrier or diluent therefor.

**3.** A pharmaceutical formulation adapted for the treatment of a condition associated with inhibiting $sPLA_2$, containing a compound as claimed in Claim 1 together with a pharmaceutically acceptable carrier or diluent therefor.

**4.** A compound as claimed in Claim 1 for use in therapy.

**5.** The use of a compound as claimed in Claim 1 for the manufacture of a medicament for treating sepsis, septic shock, rheumatoid arthritis, osteoarthritis, stroke, apoptosis, asthma, chronic bronchitis, acute bronchitis, cystic fibrosis, inflammatory bowel disease, or pancreatitis.

**6.** The use of a compound as claimed in Claim 1 for the manufacture of a medicament for alleviating the pathological

effects of sepsis, septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, Tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis; and related diseases which comprises administering to a mammal in need of such treatment a therapeutically effective amount of a compound of formula I.

**7.** The use of a compound as claimed in Claim 1 for the manufacture of a medicament for the treatment of sepsis, septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, Tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis; and related diseases.

**Patentansprüche**

**1.** Verbindung, ausgewählt aus der Gruppe, die besteht aus:

2-[4-Oxo-5-carboxamido-9-(2-methylbenzyl)-9H-pyrido[3,4-b]indolyl]essigsäure,
2-[4-Oxo-5-carboxamido-9-(3-methylbenzyl)-9H-pyrido[3,4-b]indolyl]essigsäure,
2-[4-Oxo-5-carboxamido-9-(4-methylbenzyl)-9H-pyrido[3,4-b]indolyl]essigsäure,
2-[4-Oxo-5-carboxamido-9-(4-tert-butylbenzyl)-9H-pyrido[3,4-b]indolyl]essigsäure,
2-[4-Oxo-5-carboxamido-9-pentafluorbenzyl-9H-pyrido[3,4-b]indolyl]essigsäure,
2-[4-Oxo-5-carboxamido-9-(2-fluorbenzyl)-9H-pyrido[3,4-b]indolyl]essigsäure,
2-[4-Oxo-5-carboxamido-9-(3-fluorbenzyl)-9H-pyrido[3,4-b]indolyl]essigsäure,
2-[4-Oxo-5-carboxamido-9-(4-fluorbenzyl)-9H-pyrido[3,4-b]indolyl]essigsäure,
2-[4-Oxo-5-carboxamido-9-(2,6-difluorbenzyl)-9H-pyrido[3,4-b]indolyl]essigsäure,
2-[4-Oxo-5-carboxamido-9-(3,4-difluorbenzyl)-9H-pyrido[3,4-b]indolyl]essigsäure,
2-[4-Oxo-5-carboxamido-9-(2,5-difluorbenzyl)-9H-pyrido[3,4-b]indolyl]essigsäure,
2-[4-Oxo-5-carboxamido-9-(3,5-difluorbenzyl)-9H-pyrido[3,4-b]indolyl]essigsäure,
2-[4-Oxo-5-carboxamido-9-(2,4-difluorbenzyl)-9H-pyrido[3,4-b]indolyl]essigsäure,
2-[4-Oxo-5-carboxamido-9-(2,3-difluorbenzyl)-9H-pyrido[3,4-b]indolyl]essigsäure,
2-[4-Oxo-5-carboxamido-9-[2-(trifluormethyl)benzyl)-9H-pyrido[3,4-b]indolyl]essigsäure,
2-[4-Oxo-5-carboxamido-9-[3-(trifluormethyl)benzyl)-9H-pyrido[3,4-b]indolyl]essigsäure,
2-[4-Oxo-5-carboxamido-9-[4-(trifluormethyl)benzyl)-9H-pyrido[3,4-b]indolyl]essigsäure,
2-[4-Oxo-5-carboxamido-9-[3,5-bis(trifluormethyl)benzyl)-9H-pyrido[3,4-b]indolyl]essigsäure,
2-[4-Oxo-5-carboxamido-9-[2,4-bis(trifluormethyl)benryl)-9H-pyrido[3,4-b]indolyl]essigsäure,
2-[4-Oxo-5-carboxamido-9-(α-methylnaphthyl)-9H-pyrido[3,4-b]indolyl]essigsäure,
2-[4-Oxo-5-carboxamido-9-(β-methylnaphthyl)-9H-pyrido[3,4-b]indolyl]essigsäure,

2-[4-Oxo-5-carboxamido-9-(3,5-dimethylbenzyl)-9H-pyrido[3,4-b]indolyl]essigsäure,
2-[4-Oxo-5-carboxamido-9-(2,4-dimethylbenzyl)-9H-pyrido[3,4-b]indolyl]essigsäure,
2-[4-Oxo-5-carboxamido-9-(2-phenylbenzyl)-9H-pyrido[3,4-b]indolyl]essigsäure,
2-[4-Oxo-5-carboxamido-9-(3-phenylbenzyl)-9H-pyrido[3,4-b]indolyl]essigsäure,
2-[4-Oxo-5-carboxamido-9-(4-phenylbenzyl)-9H-pyrido[3,4-b]indolyl]essigsäure,
2-[4-Oxo-5-carboxamido-9-(1-fluorenylmethyl)-9H-pyrido[3,4-b]indolyl]essigsäure,
2-[4-Oxo-5-carboxamido-9-(2-fluor-3-methylbenzyl)-9H-pyrido[3,4-b]indolyl]essigsäure,
2-[4-Oxo-5-carboxamido-9-(3-benzoylbenzyl)-9H-pyrido[3,4-b]indolyl]essigsäure,
2-[4-Oxo-5-carboxamido-9-(2-phenoxybenzyl)-9H-pyrido[3,4-b]indolyl]essigsäure,
2-[4-Oxo-5-carboxamido-9-(3-phenoxybenzyl)-9H-pyrido[3,4-b]indolyl]essigsäure,
2-[4-Oxo-5-carboxamido-9-(4-phenoxybenzyl)-9H-pyrido[3,4-b]indolyl]essigsäure,
2-[4-Oxo-5-carboxamido-9-[3-[2-(fluorphenoxy)benzyl]]-9H-pyrido[3,4-b]indolyl]essigsäure,
2-[4-Oxo-5-carboxamido-9-(3-[4-(fluorphenoxy)benzyl]]-9H-pyrido[3,4-b]indolyl]essigsäure,
2-[4-Oxo-5-carboxamido-9-[2-(fluor-3-(trifluormethyl)benzyl]-9H-pyrido[3,4-b]indolyl]essigsäure,
2-[4-Oxo-5-carboxamido-9-[2-fluor-4-(trifluormethyl)benzyl]-9H-pyrido[3,4-b]indolyl]essigsäure,
2-[4-Oxo-5-carboxamido-9-[2-fluor-5-(trifluormethyl)benzyl]-9H-pyrido[3,4-b]indolyl]essigsäure,
2-[4-Oxo-5-carboxamido-9-[3-fluor-5-(trifluormethyl)benzyl]-9H-pyrido[3,4-b]indolyl]essigsäure,
2-[4-Oxo-5-carboxamido-9-[4-fluor-2-(trifluormethyl)benryl]]-9H-pyrido[3,4 b]indolyl]essigsäure,
2-[4-Oxo-5-carboxamido-9-(4-fluor-3-(trifluormethyl)benzyl]-9H-pyrido(3,4-b]indolyl]essigsäure,
2-[4-Oxo-5-carboxamido-9-[2-fluor-6-(trifluormethyl)benzyl]-9H-pyrido[3,4-b]indolyl]essigsäure,
2-[4-Oxo-5-carboxamido-9-[2,3,6-trifluorbenzyl]-9H-pyrido[3,4-b]indolyl]essigsäure,
2-[4-Oxo-5-carboxamido-9-[2,3,5-trifluorbenzyl]-9H-pyrido[3,4-b]indolyl]essigsäure,
2-[4-Oxo-5-carboxamido-9-[2,4,5-trifluorbenzyl]-9H-pyrido[3,4-b]indolyl]essigsäure,
2-[4-Oxo-5-carboxamido-9-[2,4,6-trifluorbenzyl]-9H-pyrido[3,4-b]indolyl]essigsäure,
2-[4-Oxo-5-carboxamido-9-[2,3,4-trifluorbenzyl]-9H-pyrido[3,4-b]indolyl]essigsäure,
2-[4-Oxo-5-carboxamido-9-[3,4,5-trifluorbenzyl]-9H-pyrido[3,4-b]indolyl]essigsäure,
2-[4-Oxo-5-carboxamido-9-[3-(trifluormethoxy)benzyl]-9H-pyrido[3,4-b]indolyl]essigsäure,
2-[4-Oxo-5-carboxamido-9-[4-(trifluormethoxy)benzyl]-9H-pyrido[3,4-b]indolyl]essigsäure,
2-[4-Oxo-5-carboxamido-9-[4-methoxy(tetrafluor)benzyl]-9H-pyrido[3,4-b]indolyl]essigsäure,
2-[4-Oxo-5-carboxamido-9-(2-methoxybenzyl)-9H-pyrido[3,4-b]indolyl]essigsäure,
2-[4-Oxo-5-carboxamido-9-(3-methoxybenzyl)-9H-pyrido[3,4-b]indolyl]essigsäure,
2-[4-Oxo-5-carboxamido-9-(4-methoxybenzyl)-9H-pyrido[3,4-b]indolyl]essigsäure,
2-[4-Oxo-5-carboxamido-9-(4-ethylbenzyl)-9H-pyrido[3,4-b]indolyl]essigsäure,
2-[4-Oxo-5-carboxamido-9-(4-isopropylbenzyl)-9H-pyrido[3,4-b]indolyl]essigsäure,
2-[4-Oxo-5-carboxamido-9-(3,4,5-trimethoxybenzyl)-9H-pyrido[3,4-b]indolyl]essigsäure,
2-[4-Oxo-5-carboxamido-9-(3,4-methylendioxybenzyl)-9H-pyrido[3,4-b]indolyl]essigsäure,
2-[4-Oxo-5-carboxamido-9-(4-methoxy-3-methylbenzyl)-9H-pyrido[3,4-b]indolyl]essigsäure,
2-[4-Oxo-5-carboxamido-9-(3,5-dimethoxybenzyl)-9H-pyrido[3,4-b]indolyl]essigsäure,
2-[4-Oxo-5-carboxamido-9-(2,5-dimethoxybenzyl)-9H-pyrido[3,4-b]indolyl]essigsäure,
2-[4-Oxo-5-carboxamido-9-(4-ethoxybenzyl)-9H-pyrido[3,4-b]indolyl]essigsäure,
2-[4-Oxo-5-carboxamido-9-(cyclohexylmethyl)-9H-pyrido[3,4-b]indolyl]essigsäure,
2-[4-Oxo-5-carboxamido-9-(cyclopentylmethyl)-9H-pyrido[3,4-b]indolyl]essigsäure,
2-[4-Oxo-5-carboxamido-9-ethyl-9H-pyrido[3,4-b]indolyl]essigsäure,
2-[4-Oxo-5-carboxamido-9-(1-propyl)-9H-pyrido[3,4-b]indolyl]essigsäure,
2-[4-Oxo-5-carboxamido-9-(2-propyl)-9H-pyrido[3,4-b]indolyl]essigsäure,
2-[4-Oxo-5-carboxamido-9-(1-butyl)-9H-pyrido[3,4-b]indolyl]essigsäure,
2-[4-Oxo-5-carboxamido-9-(2-butyl)-9H-pyrido[3,4-b]indolyl]essigsäure,
2-[4-Oxo-5-carboxamido-9-isobutyl-9H-pyrido[3,4-b]indolyl]essigsäure,
2-[4-Oxo-5-carboxamido-9-[2-(1-phenylethyl)]-9H-pyrido[3,4-b]indolyl]essigsäure,
2-[4-Oxo-5-carboxamido-9-[3-(1-phenylpropyl)]-9H-pyrido[3,4-b]indolyl]essigsäure,
2-[4-Oxo-5-carboxamido-9-[4-(1-phenylbutyl)]-9H-pyrido[3,4-b]indolyl]essigsäure,
2-[4-Oxo-5-carboxamido-9-(1-pentyl)-9H-pyrido[3,4-b]indolyl]essigsäure und
2-[4-Oxo-5-carboxamido-9-(1-hexyl)-9H-pyrido[3,4-b]indolyl]essigsäure.

oder ein pharmazeutisch annehmbares Razemat, Solvat, Tautomer, optisches Isomer, Prodrugderivat hiervon, das ausgewählt ist aus dem Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyester oder ein Salz hiervon.

2. Pharmazeutische Formulierung, die eine Verbindung nach Anspruch 1 zusammen mit einem pharmazeutisch annehmbaren Träger oder Verdünnungsmittel hierfür enthält.

3. Pharmazeutische Formulierung, die zur Behandlung eines Zustands angepasst ist, der mit der Hemmung der $sPLA_2$ assoziiert ist, die eine Verbindung nach Anspruch 1 zusammen mit einem pharmazeutisch annehmbaren Träger oder Verdünnungsmittel hierfür enthält.

4. Verbindung nach Anspruch 1 zur Verwendung in der Therapie.

5. Verwendung einer Verbindung nach Anspruch 1 zur Herstellung eines Arzneimittels zur Behandlung von Sepsis, septischem Schock, rheumatoider Arthritis, Osteoarthritis, Schlaganfall, Apoptose, Asthma, chronischer Bronchitis, akuter Bronchitis, cystischer Fibrose, entzündlicher Darmerkrankung oder Pankreatitis.

6. Verwendung einer Verbindung nach Anspruch 1 zur Herstellung eines Arzneimittels zur Linderung der pathologischen Effekte von Sepsis, septischem Schock, Atemstreßsyndrom beim Erwachsenen, Pankreatitis, Trauma-induziertem Schock, Bronchialasthma, allergischer Rhinitis, rheumatoider Arthritis, cystischer Fibrose, Schlaganfall, akuter Bronchitis, chronischer Bronchitis, akuter Broncheolitis, chronischer Broncheolitis, Osteoarthritis, Gicht, Spondylarthropathie, Spondylitis ankylosans, Reiter Syndrom, psoriatischer Arthropathie, enterapathischer Spondylitis, juveniler Arthropathie oder juveniler Spondylitis ankylosans, reaktiver Arthropathie, infektiöser oder postinfektiöser Arthritis, Gonokokkenarthritis, Tuberkulosearthritis, viraler Arthritis, pilzbedingter Arthritis, Syphilis-bedingter Arthritis, Lyme-Erkrankung, Arthritis, die mit "vaskulitischen Syndromen" assoziiert ist, Polyarteriitis nodosa, hyperempfindlicher Vaskulitis, Luegenec Granulomatose, Polymyalgia rheumatica, Gelenkszellarteriitis, Arthropathie durch Calciumkristallablagerung, Pseudogicht, nicht-artikulärem Rheuma, Bursitis, Tenosynovitis, Epicondylitis (Tennisellenbogen), Carpaltunnelsyndrom, Verletzung durch wiederkehrende Tätigkeit (Tippen), verschiedenen Formen der Arthritis, neuropathischer Gelenkserkrankung (Charcotgelenk und Gelenke), Hämarthrose (hämarthrotisch), Purpura Schönlein-Hennoch, hypertropher Osteoarthropathie, multizentrischer Reticulohistiocytose, Arthritis, die mit bestimmten Erkrankungen assoziiert ist, Surcoilose, Hämochromatose, Sichelzellerkrankung und anderer Hämoglobinopathien, Hyperlipoproteinämie, Hypogammaglobulinämie, Hyperparathyreoidismus, Akromegalie, familiärem Mittelmeerfieber, Behat Erkrankung, systemischem Lupus erythematodes oder Polychondritisrückfall und verwandter Krankheiten, die die Verabreichung einer therapeutisch wirksamen Menge einer Verbindung der Formel I an einen Säuger umfasst, der einer solchen Behandlung bedarf.

7. Verwendung einer Verbindung nach Anspruch 1 zur Herstellung eines Arzneimittels zur Behandlung von Sepsis, septischem Schock, Atemstreßsyndrom beim Erwachsenen, Pankreatitis, Trauma-induziertem Schock, Bronchialasthma, allergischer Rhinitis, rheumatoider Arthritis, cystischer Fibrose, Schlaganfall, akuter Bronchitis, chronischer Bronchitis, akuter Broncheolitis, chronischer Broncheolitis, Osteoarthritis, Gicht, Spondylarthropathie, Spondylitis ankylosans, Reiter Syndrom, psoriatischer Arthropathie, enterapathischer Spondylitis, juveniler Arthropathie oder juveniler Spondylitis ankylosans, reaktiver Arthropathie, infektiöser oder postinfektiöser Arthritis, Gonokokkenarthritis, Tuberkulosearthritis, viraler Arthritis, pilzbedingter Arthritis, Syphilis-bedingter Arthritis, Lyme-Erkrankung, Arthritis, die mit "vaskulitischen Syndromen" assoziiert ist, Polyarteriitis nodosa, hyperempfindlicher Vaskulitis, Luegenec Granulomatose, Polymyalgia rheumatica, Gelenkszellarteriitis, Arthropathie durch Calciumkristallablagerung, Pseudogicht, nicht-artikulärem Rheuma, Bursitis, Tenosynovitis, Epicondylitis (Tennisellenbogen), Carpaltunnelsyndrom, Verletzung durch wiederkehrende Tätigkeit (Tippen), verschiedenen Formen der Arthritis, neuropathischer Gelenkserkrankung (Charcotgelenk und Gelenke), Hämarthrose (hämarthrotisch), Purpura Schönlein-Hennoch, hypertropher Osteoarthropathie, multizentrischer Reticulohistiocytose, Arthritis, die mit bestimmten Erkrankungen assoziiert ist, Surcoilose, Hämochromatose, Sichelzellerkrankung und anderen Hämoglobinopathien, Hyperlipoproteinämie, Hypogammaglobulinämie, Hyperparathyreoidismus, Akromegalie, familiärem Mittelmeerfieber, Behat Erkrankung, systemischem Lupus erythematodes oder Polychondritisrückfall und verwandten Krankheiten.

## Revendications

1. Composé choisi dans le groupe constitué de
l'acide 2-[4-oxo-5-carboxamido-9-(2-méthylbenzyl)-9H-pyrido[3,4-b]indolyl]-acétique ;
l'acide 2-[4-oxo-5-carboxamido-9-(3-méthylbenzyl)-9H-pyrido[3,4-b]indolyl]-acétique ;
l'acide 2-[4-oxo-5-carboxamido-9-(4-méthylbenzyl)-9H-pyrido[3,4-b]indolyl]-acétique ;
l'acide 2-[4-oxo-5-carboxamido-9-(4-tert-butylbenzyl)-9H-pyrido[3,4-b]indolyl]-acétique ;

l'acide 2-[4-oxo-5-carboxamido-9-pentafluorobenzyl-9H-pyrido[3,4-b]indolyl]-acétique ;
l'acide 2-[4-oxo-5-carboxamido-9-(2-fluorobenzyl)-9H-pyrido[3,4-b]indolyl]-acétique ;
l'acide 2-[4-oxo-5-carboxamido-9-(3-fluorobenzyl)-9H-pyrido[3,4-b]indolyl]-acétique ;
l'acide 2-[4-oxo-5-carboxamido-9-(4-fluorobenzyl)-9H-pyrido[3,4-b]indolyl]-acétique ;
l'acide 2-[4-oxo-5-carboxamido-9-(2,6-difluorobenzyl)-9H-pyrido[3,4-b]indolyl]-acétique ;
l'acide 2-[4-oxo-5-carboxamido-9-(3,4-difluorobenzyl)-9H-pyrido[3,4-b]indolyl]-acétique ;
l'acide 2-[4-oxo-5-carboxamido-9-(2,5-difluorobenzyl)-9H-pyrido[3,4-b]indolyl]-acétique ;
l'acide 2-[4-oxo-5-carboxamido-9-(3,5-difluorobenzyl)-9H-pyrido[3,4-b]indolyl]-acétique ;
l'acide 2-[4-oxo-5-carboxamido-9-(2,4-difluorobenzyl)-9H-pyrido[3,4-b]indolyl]-acétique ;
l'acide 2-[4-oxo-5-carboxamido-9-(2,3-difluorobenzyl)-9H-pyrido[3,4-b]indolyl]-acétique ;
l'acide 2-[4-oxo-5-carboxamido-9-[2-(trifluorométhyl)benzyl]-9H-pyrido[3,4-b]-indolyl]acétique ;
l'acide 2-[4-oxo-5-carboxamido-9-[3-(trifluorométhyl)benzyl]-9H-pyrido[3,4-b]-indolyl]acétique ;
l'acide 2-[4-oxo-5-carboxamido-9-[4-(trifluorométhyl)benzyl]-9H-pyrido[3,4-b]-indolyl]acétique ;
l'acide 2-[4-oxo-5-carboxamido-9-[3,5-bis(trifluorométhyl)benzyl]-9H-pyrido-[3,4-b]indolyl]acétique ;
l'acide 2-[4-oxo-5-carboxamido-9-[2,4-bis(trifluorométhyl)benzyl]-9H-pyrido-[3,4-b]indolyl]acétique ;
l'acide 2-[4-oxo-5-carboxamido-9-(a-méthylnaphtyl)-9H-pyrido[3,4-b]indolyl]-acétique ;
l'acide 2-[4-oxo-5-carboxamido-9-(b-méthylnaphtyl)-9H-pyrido[3,4-b]indolyl]-acétique ;
l'acide 2-[4-oxo-5-carboxamido-9-(3,5-diméthylbenzy)-9H-pyrido[3,4-b]indolyl]-acétique ;
l'acide 2-[4-oxo-5-carboxamido-9-(2,4-diméthylbenzyl)-9H-pyrido[3,4-b]indolyl]-acétique ;
l'acide 2-[4-oxo-5-carboxamido-9-(2-phénylbenzyl)-9H-pyrido[3,4-b]indolyl]-acétique ;
l'acide 2-[4-oxo-5-carboxamido-9-(3-phénylbenzyl)-9H-pyrido[3,4-b]indolyl]-acétique ;
l'acide 2-[4-oxo-5-carboxamido-9-(4-phénylbenzyl)-9H-pyrido[3,4-b]indolyl]-acétique ;
l'acide 2-[4-oxo-5-carboxamido-9-(1-fluorénylméthyl)-9H-pyrido[3,4-b]indolyl]-acétique ;
l'acide 2-[4-oxo-5-carboxamido-9-(2-fluoro-3-méthylbenzyl)-9H-pyrido[3,4-b]-indolyl] acétique ;
l'acide 2-[4-oxo-5-carboxamido-9-(3-benzoylbenzyl)-9H-pyrido[3,4-b]indolyl]-acétique ;
l'acide 2-[4-oxo-5-carboxamido-9-(2-phénoxybenzyl)-9H-pyrido[3,4-b]indolyl]-acétique ;
l'acide 2-[4-oxo-5-carboxamido-9-(3-phénoxybenzyl)-9H-pyrido[3,4-b]indolyl]-acétique ;
l'acide 2-[4-oxo-5-carboxamido-9-(4-phénoxybenzyl)-9H-pyrido[3,4-b]indolyl]-acétique ;
l'acide 2-[4-oxo-5-carboxamido-9-[3-[2-(fluorophénoxy)benzyl]]-9-pyrido[3,4-b]-indolyl]acétique ;
l'acide 2-[4-oxo-5-carboxamido-9-[3-[4-(fluorophénoxy)benzyl]]-9Hpyrido[3,4-b]-indolyl]acétique ;
l'acide 2-[4-oxo-5-carboxamido-9-[2-fluoro-3-(trifluorométhyl)benzyl]-9H-pyrido-[3,4-b]indolyl]acétique ;
l'acide 2-[4-oxo-5-carboxamido-9-[2-fluoro-4-(trifluorométhyl)benzyl]-9H-pyrido-[3,4-b]indolyl]acétique ;
l'acide 2-[4-oxo-5-carboxamido-9-[2-fluoro-5-(trifluorométhyl)benzyl]-9H-pyrido-[3,4-b]indolyl]acétique ;
l'acide 2-[4-oxo-5-carboxamido-9-[3-fluoro-5-(trifluorométhyl)benzyl]-9H-pyrido-[3,4-b]indolyl]acétique ;
l'acide 2-[4-oxo-5-carboxamido-9-[4-fluoro-2-(trifluorométhyl)benzyl]-9H-pyrido-[3,4-b]indolyl]acétique ;
l'acide 2-[4-oxo-5-carboxamido-9-[4-fluoro-3-(trifluorométhyl)benzyl]-9H-pyrido-[3,4-b]indolyl]acétique ;
l'acide 2-[4-oxo-5-carboxamido-9-[2-fluoro-6-(trifluorométhyl)benzyl]-9H-pyrido-[3,4-b]indolyl]acétique ;
l'acide 2-[4-oxo-5-carboxamido-9-(2,3,6-trifluorobenzyl)-9H-pyrido[3,4-b]indolyl]-acétique ;
l'acide 2-[4-oxo-5-carboxamido-9-(2,3,5-trifluorobenzyl)-9H-pyrido[3,4-b]indolyl]-acétique ;
l'acide 2-[4-oxo-5-carboxamido-9-(2,4,5-trifluorobenzyl)-9H-pyrido(3,4-b]indolyl]-acétique ;
l'acide 2-[4-oxo-5-carboxamido-9-(2,4,6-trifluorobenzyl)-9H-pyrido[3,4-b]indolyl]-acétique ;
l'acide 2-[4-oxo-5-carboxamido-9-(2,3,4-trifluorobenzyl)-9H-pyrido[3,4-b]indolyl]-acétique ;
l'acide 2-[4-oxo-5-carboxamido-9-(3,4,5-trifluorobenzyl)-9H-pyrido[3,4-b]indolyl]-acétique ;
l'acide 2-[4-oxo-5-carboxamido-9-[3-(trifluorométhoxyl)benzyl]-9H-pyrido[3,4-b]-indolyl]acétique ;
l'acide 2-[4-oxo-5-carboxamido-9-[4-(trifluorométhoxyt)benzyl]-9H-pyrido[3,4-b]-indolyl]acétique ;
l'acide 2-[4-oxo-5-carboxamido-9-[4-méthoxy(tétrafluoro)benzyl]-9H-pyrido-[3,4-b]indolyl]acétique ;
l'acide 2-[4-oxo-5-carboxamido-9-(2-méthoxybenzyl)-9H-pyrido[3,4-b]indolyl]-acétique ;
l'acide 2-[4-oxo-5-carboxamido-9-(3-méthoxybenzyl)-9H-pyrido[3,4-b]indolyl]-acétique ;
l'acide 2-[4-oxo-5-carboxamido-9-(4-méthoxybenzyl)-9H-pyrido[3,4-b]indolyl]-acétique ;
l'acide 2-[4-oxo-5-carboxamido-9-(4-éthylbenzyl)-9H-pyrido[3,4-b]indolyl]-acétique,
l'acide 2-[4-oxo-5-carboxamido-9-(4-isopropylbenzyl)-9H-pyrido[3,4-b]indolyl]-acétique ;
l'acide 2-[4-oxo-5-carboxamido-9-(3,4,5-triméthoxybenzyl)-9H-pyrido[3,4-b]-indolyl]acétique ;
l'acide 2-[4-oxo-5-carboxamido-9-(3,4-méthylènedioxybenzyl)-9H-pyrido[3,4-b]-indolyl]acétique ;
l'acide 2-[4-oxo-5-carboxamido-9-(4-méthoxy-3-méthylbenzyl)-9H-pyrido[3,4-b]-indolyl]acétique ;
l'acide 2-[4-oxo-5-carboxamido-9-(3,5-diméthoxybenzyl)-9H-pyrido[3,4-b]-indolyl]acétique ;
l'acide 2-[4-oxo-5-carboxamido-9-(2,5-diméthoxybenzyl)-9H-pyrido[3,4-b]-indolyl]acétique ;
l'acide 2-[4-oxo-5-carboxamido-9-(4-éthoxybenzyl)-9H-pyrido[3,4-b]indolyl]-acétique ;
l'acide 2-[4-oxo-5-carboxamido-9-(cyclohexylméthyl)-9H-pyrido[3,4-b]indolyl]-acétique ;

l'acide 2-[4-oxo-5-carboxamido-9-(cyclopentylméthyl)-9H-pyrido[3,4-b]indolyl]-acétique ;
l'acide 2-[4-oxo-5-carboxamido-9-éthyl-9H-pyrido[3,4-b]indolyl]acétique ;
l'acide 2-[4-oxo-5-carboxamido-9-(1-propyl)-9H-pyrido[3,4-b]indolyl]acétique ;
l'acide 2-[4-oxo-5-carboxamido-9-(2-propyl)-9H-pyrido[3,4-b] indolyl]acétique ;
l'acide 2-[4-oxo-5-carboxamido-9-(1-butyl)-9H-pyrido[3,4-b]indolyl]acétique ;
l'acide 2-[4-oxo-5-carboxamido-9-(2-butyl)-9H-pyrido[3,4-b]indolyl]acétique ;
l'acide 2-[4-oxo-5-carboxamido-9-isobutyl-9H-pyrido[3,4-b]indolyl]acétique ;
l'acide 2-[4-oxo-5-carboxamido-9-[2-(1-phényléthyl)]-9H-pyrido[3,4-b]indolyl]-acétique ;
l'acide 2-[4-oxo-5-carboxamido-9-[3-(1-phénylpropyl)]-9H-pyrido[3,4-b]indolyl]-acétique ;
l'acide 2-[4-oxo-5-carboxamido-9-[4-(1-phénylbutyl)]-9H-pyrido[3,4-b]indolyl]-acétique ;
l'acide 2-[4-oxo-5-carboxamido-9-(1-pentyl)-9H-pyrido[3,4-b]indolyl]acétique ;
et l'acide 2-[4-oxo-5-carboxamido-9-(1-hexyl)-9H-pyrido[3,4-b]indolyl]acétique ou un racémate, un solvate, un tautomère, un isomère optique pharmaceutiquement acceptable, un précurseur d'un médicament choisi parmi l'ester méthylique, l'ester éthylique, l'ester propylique, l'ester isopropylique, l'ester butylique, l'ester sec-butylique, l'ester tert-butylique, l'ester morpholinoéthyloxy, l'ester diéthylglycolamide ou l'ester diéthylaminocarbonylméthoxy de ceux-ci ou un sel de ceux-ci.

**2.** Formulation pharmaceutique comprenant un composé tel que revendiqué dans la revendication 1 avec un support ou un diluant pharmaceutiquement acceptable pour celui-ci.

**3.** Formulation pharmaceutique adaptée pour le traitement d'un état associé à l'inhibition de la $sPLA_2$, contenant un composé tel que revendiqué dans la revendication 1 avec un support ou un diluant pharmaceutiquement acceptable pour celui-ci.

**4.** Composé tels que revendiqué dans la revendication 1 pour une utilisation en thérapie.

**5.** Utilisation d'un composé tel que revendiqué dans la revendication 1 pour la préparation d'un médicament pour traiter la septicémie, le choc septique, l'arthrite rhumatoïde, l'ostéoarthrite, une attaque, l'apoptose, l'asthme, la bronchite chronique, la bronchite aiguë, la fibrose cystique, la maladie de l'intestin inflammatoire ou la pancréatite.

**6.** Utilisation d'un composé tel que revendiqué dans la revendication 1 pour la préparation d'un médicament pour soulager les effets pathologiques d'une septicémie, d'un choc septique, du syndrome de détresse respiratoire adulte, de la pancréatite, du choc induit par un traumatisme, de l'asthme bronchique, de la rhinite allergique, de l'arthrite rhumatoïde, de la fibrose cystique, d'une attaque, de la bronchite aiguë, de la bronchite chronique, de la bronchiolyte aiguë, de la bronchiolyte chronique, de l'ostéoarthrite, de la goutte, de la spondylarthropathie, de la spondylite ankylosante, du syndrome de Reiter, de l'arthropathie psoriatique, de la spondylite entéropathique, de l'arthropathie juvénile ou de la spondylite ankylosante juvénile, de l'arthropathie réactive, de l'arthrite infectieuse ou post-infectieuse, de l'arthrite gonococcique, de l'arthrite tuberculeuse, de l'arthrite virale, de l'arthrite fongique, de l'arthrite syphilitique, de la maladie de Lyme, de l'arthrite associée à des syndromes vascularitiques, de la périartérite noueuse, de la vascularite d'hypersensibilité, de la granulomatose de Luegenec, de la pseudopolyarthrite rhizomélique, de l'artérite articulaire à cellules, de l'arthropathie par dépôt de cristaux de calcium, de la pseudo-goutte, du rhumatisme non articulaire, de la bursite, de la ténosynovite, de l'épicondylite (tennis elbow), du syndrome du canal carpien, des lésions dues à une utilisation répétitive (dactylographie), de différentes formes d'arthrite, de la maladie neuropathique des articulations (charco et articulation), de l'hémarthrose, du purpura de Henoch-Schonlein, de l'ostéoarthropathie hypertrophique, de la réticulohistiocytose multicentrique, de l'arthrite associée à certaines maladies, de la surcoilose, de l'hémochromatose, de la drépanocytose et d'autres hémoglobinopathies, de l'hyperlipoprotéinémie, de l'hypogammaglobulinémie, de l'hyperparathyroïdie, de l'acromégalie, de la brucellose, de la maladie de Behat, du lupus érythémateux systémique ou de la polychondrite chronique et des maladies associées, qui comprend l'administration à un mammifère qui a besoin d'un tel traitement d'une quantité thérapeutiquement efficace d'un composé de formule I.

**7.** Utilisation d'un composé tel que revendiqué dans la revendication 1 pour la préparation d'un médicament pour le traitement d'une septicémie, d'un choc septique, du syndrome de détresse respiratoire adulte, de la pancréatite, du choc induit par un traumatisme, de l'asthme bronchique, de la rhinite allergique, de l'arthrite rhumatoïde, de la fibrose cystique, d'une attaque, de la bronchite aiguë, de la bronchite chronique, de la bronchiolyte aiguë, de la bronchiolyte chronique, de l'ostéoarthrite, de la goutte, de la spondylarthropathie, de la spondylite ankylosante, du syndrome de Reiter, de l'arthropathie psoriatique, de la spondylite entéropathique, de l'arthropathie juvénile ou de la spondylite ankylosante juvénile, de l'arthropathie réactive, de l'arthrite infectieuse ou post-infectieuse, de

l'arthrite gonococcique, de l'arthrite tuberculeuse, de l'arthrite virale, de l'arthrite fongique, de l'arthrite syphilitique, de la maladie de Lyme, de l'arthrite associée à des syndromes vascularitiques, de la périartérite noueuse, de la vascularite d'hypersensibilité, de la granulomatose de Luegenec, de la pseudopolyarthrite rhizomélique, de l'artérite articulaire à cellules, de l'arthropathie par dépôt de cristaux de calcium, de la pseudo-goutte, du rhumatisme non articulaire, de la bursite, de la ténosynovite, de l'épicondylite (tennis elbow), du syndrome du canal carpien, des lésions dues à une utilisation répétitive (dactylographie), de différentes formes d'arthrite, de la maladie neuropathique des articulations (charco et articulation), de l'hémarthrose, du purpura de Henoch-Schonlein, de l'ostéoarthropathie hypertrophique, de la réticulohistiocytose multicentrique, de l'arthrite associée à certaines maladies, de la surcoilose, de l'hémochromatose, de la drépanocytose et d'autres hémoglobinopathies, de l'hyperlipoprotéinémie, de l'hypogammaglobulinémie, de l'hyperparathyroïdie, de l'acromégalie, de la brucellose, de la maladie de Behat, du lupus érythémateux systémique ou de la polychondrite chronique et des maladies associées.